(19) Europäisches Patentamt
European Patent Office
Office européen des brevets

(11) **EP 4 475 755 B1**

(12) **EUROPEAN PATENT SPECIFICATION**

(45) Date of publication and mention
of the grant of the patent:
**12.11.2025 Bulletin 2025/46**

(21) Application number: **23703485.5**

(22) Date of filing: **10.02.2023**

(51) International Patent Classification (IPC):
*A61B 5/11* (2006.01)    *A61B 5/00* (2006.01)
*A63B 24/00* (2006.01)

(52) Cooperative Patent Classification (CPC):
**A61B 5/1102; A61B 5/1118; A61B 5/4866;**
A61B 2562/0219

(86) International application number:
**PCT/EP2023/053302**

(87) International publication number:
**WO 2023/152288 (17.08.2023 Gazette 2023/33)**

(54) **SYSTEM AND METHOD FOR MEASUREMENT OF CARDIORESPIRATORY FITNESS OF A SUBJECT**

SYSTEM UND VERFAHREN ZUR MESSUNG DER KARDIORESPIRATORISCHEN FITNESS EINES SUBJEKTS

SYSTÈME ET PROCÉDÉ DE MESURE DE L'APTITUDE CARDIORESPIRATOIRE D'UN SUJET

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.02.2022 EP 22156055**

(43) Date of publication of application:
**18.12.2024 Bulletin 2024/51**

(73) Proprietor: **Université Libre de Bruxelles
1050 Bruxelles (BE)**

(72) Inventors:
• **RABINEAU, Jérémy
1190 Forest (BE)**
• **MIGEOTTE, Pierre-François
1332 Genval (BE)**
• **HOSSEIN, Amin
1070 Anderlecht (BE)**
• **QUESTEL, Philippe
1190 Bruxelles (BE)**

(74) Representative: **De Clercq & Partners
Edgard Gevaertdreef 10a
9830 Sint-Martens-Latem (BE)**

(56) References cited:
EP-A1- 3 748 641    US-A1- 2008 161 653
US-A1- 2017 156 641    US-A1- 2018 214 030
US-A1- 2018 368 737

## Description

### Field

Provided are systems and methods for measurement of maximal oxygen consumption of a subject.

### Background

Cardiorespiratory fitness represents an important aspect of a subject's physical condition and is a key indicator of a medical condition of the subject. The measurement of cardiorespiratory fitness is useful in many different situations, such as improving performance in elite athletes, monitoring a cardiac patient undergoing a rehabilitation program, monitoring cardiovascular system of deconditioning astronauts while in space, etc. The gold standard measurement for cardiorespiratory fitness assessment is the maximal oxygen uptake rate one can withstand, and more commonly known as the $VO_2$ max measurement. Obtaining an accurate $VO_2$ max measurement requires a maximal effort ergospirometry test. The test involves complex gas exchangers and heavy machinery, requires expertise and so is costly, and not always possible in all environments (e.g., sports centres, developing countries, local clinics, space vehicles).

EP3748641 A1 relates to the quantifying of cardiorespiratory fitness. It includes the obtaining of a seismocardiogram (SCG) recorded with an accelerometer configured to measure accelerations and vibrations of the chest wall of a person caused by myocardial movement. Properties of a first signal feature (AC) in the seismocardiogram (SCG) are determined, wherein the first signal feature (AC) corresponds to the aortic valve closure (AC) of a heartbeat. A measure indicating cardiorespiratory fitness ($VO_2$max) is then determined based on the properties of first signal feature (AC).

An aim to provide an affordable, light-weight, and operationally more efficient solution to the problem of cardiorespiratory fitness measurement, that offers a high level of measurement accuracy.

### Figure legends

**FIG. 1** shows exemplary placements of the motion sensing module on the posterior side (Panel A) and anterior side (Panel B), placement of the ballistocardiograph (**Panel C**) and placement of the seismocardiograph (**Panel D**) on the subject.

**FIG. 2A** shows an exemplary schematic of the device, and sensor unit containing motion sensing module, ballistocardiograph, and seismocardiograph.

**FIG. 2B** shows an exemplary schematic of the device, and sensor unit containing ballistocardiograph, and seismocardiograph.

**FIG. 3** shows in **Panel A** alternating exertion segments (S) and non-exercise periods (N) within an exercise session (E); **Panel B** examples of channel outputs from the sensor unit.

**FIG. 4** is an exemplary schematic representation of the method described herein, wherein a set of periods of mean kinetic energies (SPMKE) is transformed into a linear VO2 function (LVO2F).

**FIG. 5** is similar to FIG. 4, showing transformation of a set of periods of mean kinetic energies (SPMKE) into a linear VO2 function (LVO2F) via a set of order statistics.

**FIG. 6** shows in **Panel** A a period of mean kinetic energies (PMKE), each mean kinetic energies (MKE) determined from a different time window (W) of the kinetic energy data stream (KEDS) within a non-exercise period (N1). **Panel B** depicts the period of mean kinetic energies (PMKE) plotted as a graph of mean kinetic energies (MKE) *vs* time within the non-exercise period (N). **Panel C** shows a transformation of the set of periods of mean kinetic energies (SPMKE) into a VO2 function (VO2F) for the subject.

**FIG. 7** depicts one version of the described method in which each period of mean kinetic energies (PMKE) is transformed into an intermediate linear function (ILF), and then an order statistic (OS) of each ILF is determined, and the set of order statistics (SOS) for the exercise period is transformed into a linear VO2 function (LVO2F).

**FIGs. 8A to 8D** depict in more detail the schematic of FIG. 7: **FIG. 8A** period of mean kinetic energies (PMKE) for one non-exercise period; **FIG. 8B** transformation of the period of mean kinetic energies (PMKE) into an intermediate linear function for the non-exercise period;

**FIG. 8C** order statistics (1st quartile, median, 3rd quartile) for the intermediate linear function of the non-exercise period (N); **FIG. 8D** set of order statistics (SOS) for the exercise session (E) transformed into a linear VO2 function for the subject.

**FIG. 9** depicts one version of the described method in which an order statistic (OS) of each period of mean kinetic energies (PMKE) is determined, and then set of order statistics (SOS) for the exercise period is transformed into an intermediate linear function (ILF), then the intermediate linear function (ILF) is transformed into a linear VO2 function (LVO2F) for the subject.

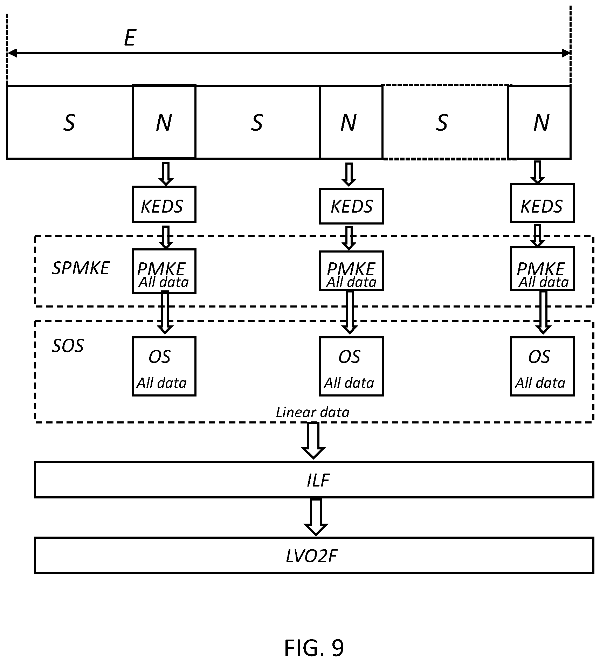

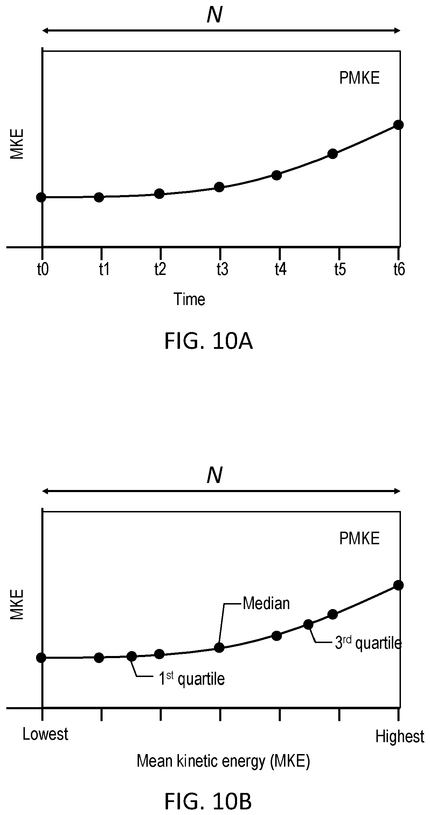

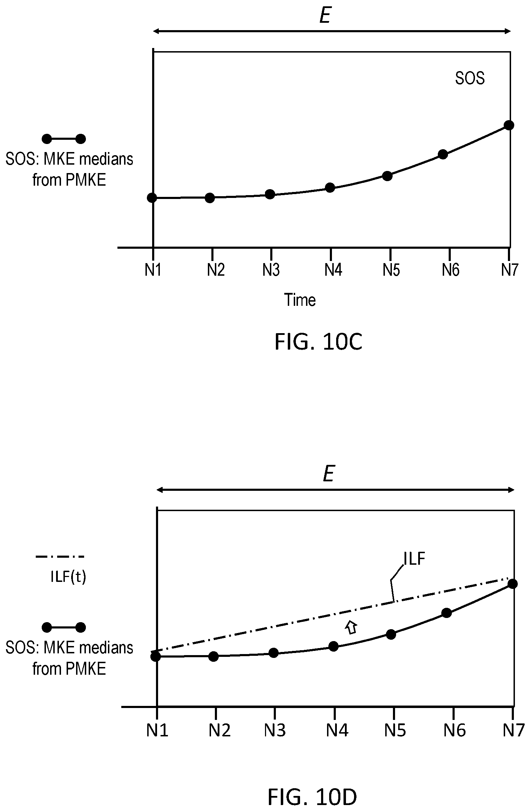

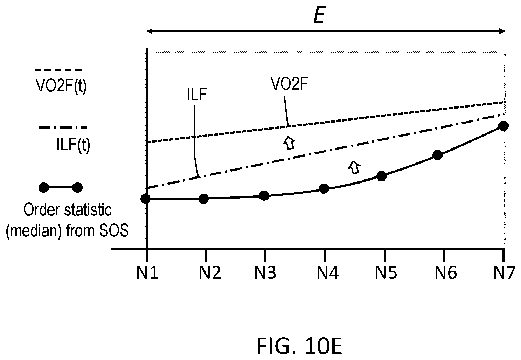

**FIGs. 10A to 10E** depicts in more detail the schematic of FIG. 9: **FIG. 10A** period of mean kinetic energies (PMKE) for one non-exercise period; **FIG. 10B** order statistics (1st quartile, median, 3rd quartile) for each PMKE of the non-exercise period (N); **FIG. 10C** order statistics (OS) (medians) of PMKEs for the exercise session (E); **FIG. 10D** transformation of the set of order statistics into an intermediate linear function (ILF) for the exercise session (E); **FIG. 10E** intermediate linear function (ILF) for the exercise session (E) transformed into a linear VO2 function for the subject.

**FIG. 11A** shows exemplary channel outputs of the sensor unit containing two motion sensing modules.

**FIG. 11B** shows exemplary channel outputs of the sensor unit containing two motion sensing modules, one being a seismocardiograph, the other being a ballistocardiograph.

**FIG. 12** Evolution of the median (full circles) and quartiles (ticks) of SOS (white) and $VO_2$ (black) over a protocol E1 for a given subject.

**FIG. 13** Scatter plot of the estimated versus measured values of VO2 max. Here, the estimates are based on prior knowledge of the maximum workload achieved and the value of the correction factor 'd'. The identity line is drawn with a solid black line, while the 10% and 20% error lines are drawn in dashed and dot-dashed lines, respectively.

## Summary

Provided herein is a system (100) for determining a cardiorespiratory fitness measurement of a subject (50), comprising a processing unit (160), the processing unit (160) configured to:

- receive a stream of signals (120) outputted by a sensor unit (110) comprising one or more motion sensing modules (111), MSMs,
- determine, from the signal stream (120), a kinetic energy data stream, KEDS, for each non-exercise period (N) of an exercise session (E) for gradually increasing the oxygen consumption, $VO_2$, of the subject, wherein the exercise session (E) comprises a plurality of alternating exertion segments (S) and non-exercise periods (N),
- for each non-exercise period (N):

  - obtain a mean kinetic energy, MKE, that is a time-averaged kinetic energy value from a time window (W) of the kinetic energy data stream, KEDS;
  - determine a period of mean kinetic energies, PMKEs, containing multiple MKEs for different time windows (W) within the non-exercise period (N);

- determine, from a set of PMKEs, SPMKE, containing multiple PMKEs within the exercise period (E), the cardiorespiratory fitness measurement of the subject.

The cardiorespiratory fitness measurement of the subject may be determined from the SPMKE by:

- transforming the SPMKE into a linear VO2 function (LVO2F), representative of an oxygen consumption, $VO_2$, of the subject.

The transformation of the SPMKE into the LVO2F, may comprise a step of converting each PMKE of the SPMKE into a scalar value representative of the non-exercise period (N), wherein the scalar value is an order statistic.

The conversion of each PMKE of the SPMKE into a scalar value may be performed:

- indirectly on the PMKE, by transforming the MKE values within the PMKE first into an intermediate linear function (ILF), and then by value-ordering the values within the ILF to obtain the order statistic, or
- directly on the PMKE, by value-ordering the MKE values within the PMKE to obtain the order statistic.

The same order statistic may be determined for each non-exercise period (N), at least some of these order statistics (OS) form a set of order statistics, SOS, for the exercise session (E), and the SOS is transformed into the LVO2F of the subject.

The transformation of the SOS into the LVO2F may be performed:

- directly on the SOS, or
- indirectly on the SOS, by first transforming the order statistics (OS) values within the SOS into an intermediate linear function (ILF), and then transforming the intermediate linear function (ILF) into the LVO2F of the subject.

The order statistic (OS) may be either a median or a quantile, and the order statistics (OS) within each set of order statistics (SOS) are either all medians or all quantiles.

The exercise session (E) may bring the subject to a level of exhaustion, preferably to a level of full exhaustion.

The time window (W) may be for a period of w seconds, and the value of w is greater than a duration of 1 cardiac cycle of the subject.

The value of w may be the same during each non-exercise period (N) and during the exercise session (E).

The cardiorespiratory fitness measurement of the subject (50) may be a $VO_2$ max measurement of the subject (50).

One of the MSM (111) may be a seismocardiograph (114), SCG, and optionally a further MSM (111) may be a ballistocardiograph (112), BCG.

Further provided is a computer-implemented method for determining a cardiorespiratory fitness measurement of a subject (50), comprising:

- receiving a stream of signals (120) outputted by a sensor unit (110) comprising one or more motion sensing modules (111),
- determining, from the signal stream (120), a kinetic energy data stream, KEDS, for each non-exercise period (N) of an exercise session (E) for gradually increasing the oxygen consumption, $VO_2$, of the subject, wherein the exercise session (E) comprises a plurality of alternating exertion segments (S) and non-exercise periods (N),
- for each non-exercise period (N):

  - obtaining a mean kinetic energy (MKE) that is a time-averaged kinetic energy value from a time window (W) of the kinetic energy data stream, KEDS;
  - determining multiple mean kinetic energies (MMKEs), for different time windows (W);
  - determining an order statistic (OS) from the multiple mean kinetic energies, (MMKEs)

- determining a set of order statistics (SOS) containing at least some of the order statics (OS) determined from the plurality of non-exercise periods (N) of the exercise session (E);
- determine from the set of order statistics (SOS), the cardiorespiratory fitness measurement of the subject.

The computer-implemented method may comprise a limitation as defined herein, in particular above.

Further provided is a computer program or computer program product having instructions which when executed by a computing device or system cause the computing device or system to perform the computer-implemented method as defined herein.

**Detailed description**

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any $\geq 3, \geq 4, \geq 5, \geq 6$ or $\geq 7$ etc. of said members, and up to all said members.

The term "each" means an individual value or group of values within a set. It does not necessarily mean each and every value within the set.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

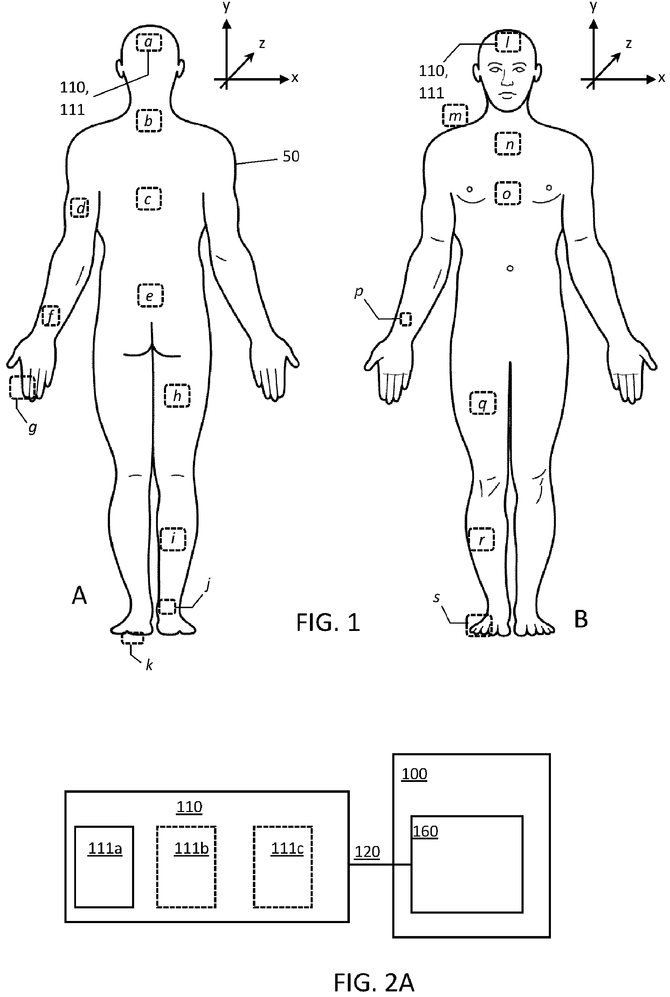

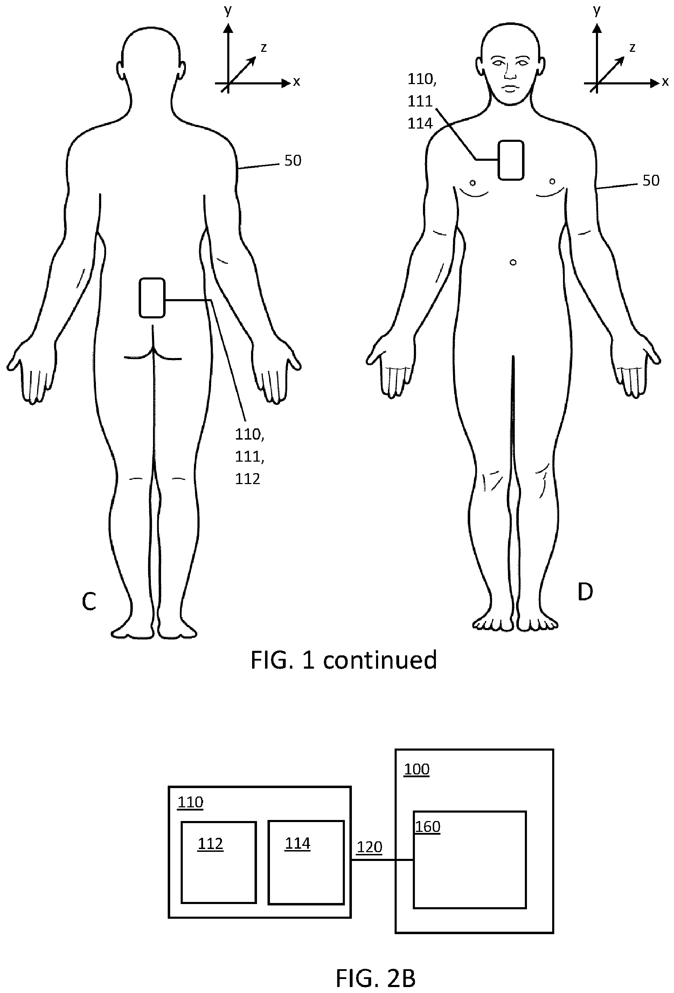

Provided is a system (100) and method for determining a cardiorespiratory fitness measurement, in particular, a maximal oxygen consumption, $VO_2$ max, of a subject (50). An exemplary system (100) is shown in **FIG. 2A** and **FIG.** 2B.

The method or system comprising a processing unit (160) configured to receive a stream of signals (120) outputted by a sensor unit (110). The sensor unit (110) comprises one or more motion sensing modules (MSMs) (111), each motion sensing module (MSM) being configured for detection of bodily vibrations induced by cardiovascular activity within the body. The sensor unit (110) may comprise a motion sensing module (MSM) (111) that is a seismocardiograph (SCG) (114), and optionally a motion sensing module (MSM) (111) that is a ballistocardiograph (BCG) (112), and optionally one or more electrocardiograph, ECG, electrodes.

The method or processing unit (160) determines, from the signal stream (120), a kinetic energy data stream, KEDS, for each non-exercise period (N) of an exercise session (E) for gradually increasing the oxygen consumption, $VO_2$, of the subject, wherein the exercise session (E) comprises a plurality of alternating exertion segments (S) and non-exercise periods (N).

The method or processing unit (160) is further configured to, for each non-exercise period (N):

- obtain a mean kinetic energy, MKE, that is a time-averaged kinetic energy value from a time window (W) of the kinetic energy data stream, KEDS;
- determine a period mean kinetic energies, PMKEs, containing a plurality of MKEs for different time windows (W).

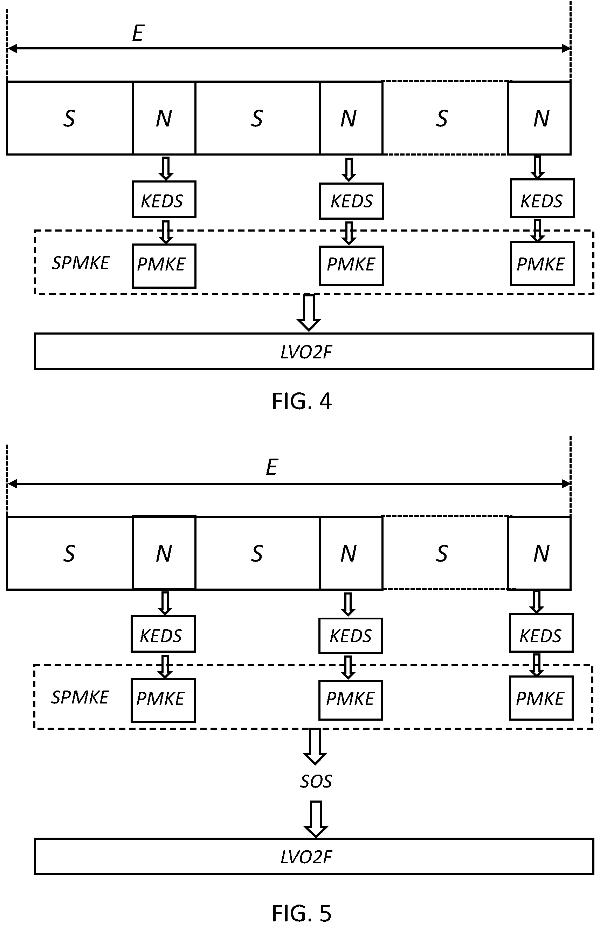

The method or processing unit (160) is further configured to obtain a set of PMKEs (SPMKE), wherein a SPMKE contains a plurality of PMKEs (periods of mean kinetic energy) obtained during the course of the exercise session (E). The cardiorespiratory fitness measurement of the subject can be determined from the set of PMKEs (SPMKE), as the SPMKE is indicative of the oxygen consumption rate ($VO_2$) over time for the exercise session (E). The steps are shown in **FIG. 4**. In particular, the $VO_2$ max for the subject, can be determined from the oxygen consumption rate ($VO_2$) corresponding to a time point at which the subject reaches or is predicted to reach exhaustion.

The cardiorespiratory fitness measurement of a subject is a measure of a subject's physical condition. In particular, it is a measure of the maximal rate of oxygen consumption rate (by volume) under maximal exertion by the subject. In other words, the more oxygen the subject is able to consume, the greater amount of oxygen arrives in the muscle cells, allowing the subject to withstand longer and/or more intensive exercise. Maximal oxygen uptake can be detected if an increase in power (for example the resistance on a bike exercise) is not met by an increase in oxygen consumption rate. This means that the body is no longer capable of increasing its oxygen consumption and enters the anaerobic phase (energy consumption in the absence of oxygen). $VO_2$ max is one measurement of cardiorespiratory fitness.

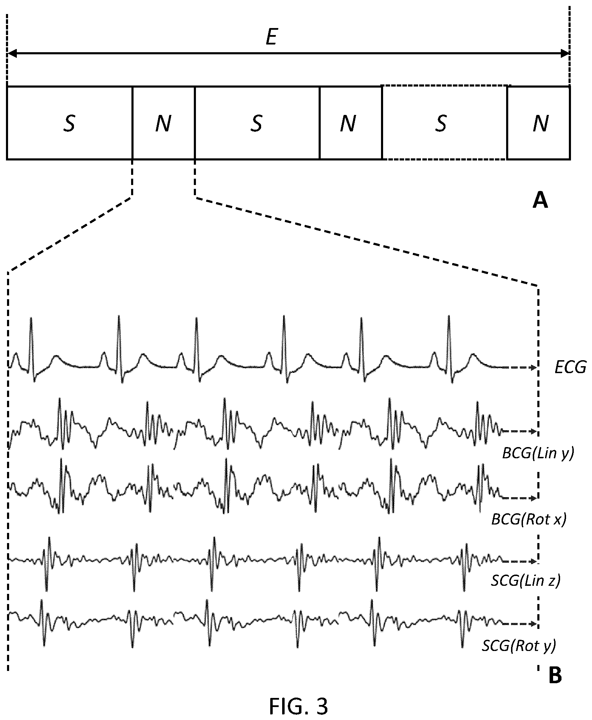

An exercise session (E) undertaken by the subject contains a plurality of alternating exertion segments (S) and non-exercise periods (N). The exercise session (E) gradually increases the $VO_2$ of the subject. An example of an exercise session (E) is shown in **FIG. 3** to **5, 7,** and 9; there is a plurality of exertion segments (S) and subsequent rest periods (non-exercise (N) periods). The exercise session (E) gradually increases the heart rate and hence oxygen consumption ($VO_2$) of the subject. The exercise session (E) brings the subject to a level of exhaustion. The subject may continue to exercise until they can no longer continue (full exhaustion). The subject may continue to exercise until they reach a pre-determined level of exhaustion, e.g., 16 to 19 on the Borg scale (rating of perceived exertion (RPE) as known in the art). The duration of the exercise session (E) is determined by the level of exhaustion of the subject.

The exertion segment (S) may involve using a stationary exercise apparatus such as a bicycle, elliptical trainer, treadmill.

The intensity of the exertion segment (S) is set to increase the heart and respiration rates. The intensity of the exertion segment (S) may be between 50 and 300 W, *i.e.,* the subject expends this amount of energy during the exertion segment (S). The intensity of the exertion segment (S) may be the same for each exertion segment (S) throughout the exercise session (E). The intensity of an exertion segment (S) may gradually increase as the exercise session (E) progresses.

The length of an exertion segment (S) may be 30 to 180 seconds, more preferably 60 to 180 seconds. The length of an exertion segment (S) may gradually increase as the exercise session (E) progresses. Preferably, the length of the exertion segment (S) is the same for each exertion segment (S) throughout the exercise session (E).

The first exertion segment (S) may be a warm-up session designed to gently raise the heart and respiration rates. The intensity of the warm-up session may be lower than in subsequent exertion segments (S). The length of the warm-up session may be longer than in subsequent exertion segments (S).

The number of exertion segments (S) within the exercise session (E) may vary from subject to subject and, as mentioned elsewhere, is determined by a level of exhaustion of the subject. The subject may continue to exercise until they could no longer continue (full exhaustion), or until they reach a pre-determined level of exhaustion, *e.g.*, 16 to 19 on the Borg scale (rating of perceived exertion (RPE)).

The non-exercise period (N) is a period of non-activity. During the non-exercise period (N) the subject may sit, stand up, or lie down. The non-exercise period (N) typically follows an exertion segment (S); in such case it is a rest or recovery period.

The length of a non-exercise period (N) may be 20 to 60 seconds, more preferably 30 to 45 seconds. Preferably, the length of the non-exercise period (N) is the same for each non-exercise period (N) throughout the exercise session (E).

The period prior to the first exertion segment (S) is a baseline period in which a baseline measurement is taken of the subject; this may or may not be used as non-exercise period (N) for determining the cardiorespiratory fitness, in particular, $VO_2$ max.

The period after the first exertion segment (warm-up session) may or may not be used as non-exercise period (N) for determining the cardiorespiratory fitness measurement, in particular, the $VO_2$ max. Preferably it is used as non-exercise period (N) for determining the cardiorespiratory fitness measurement, in particular, the $VO_2$ max.

The sensor unit (110) comprises one or more motion sensing modules (MSMs) (111). It may further optionally comprise one or more electrocardiograph (ECG) electrodes.

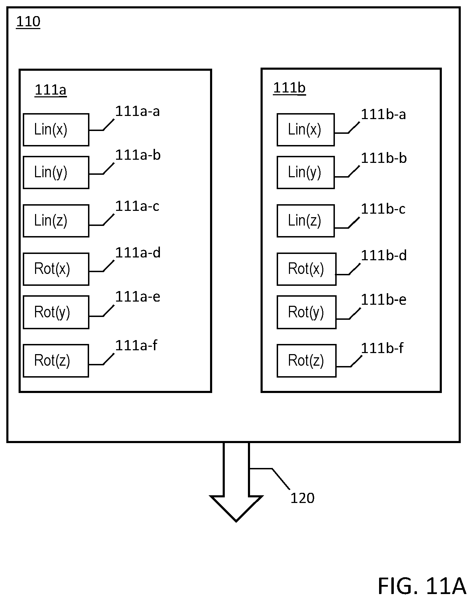

The sensor unit (110) outputs a signal stream (120) from one of more movement sensors in the one or more motion sensing modules (MSMs) (111). The ECG output may or may not be part of the signal stream (120). **FIG. 2A** shows an example of a sensor unit (110) containing at least one motion sensing module (MSM) (111a) and optionally two further motion sensing modules (MSMs) (111b, 111c), sending a signal stream (120) to the processing unit (160). In particular, sensor unit (110) outputs a signal stream (120) containing one or more channel outputs generated by the motion sensing modules (MSM). A channel output reports either a linear or rotation movement (acceleration or velocity) in one direction (x, y, z). An example of the sensor unit (110), and possible multiple channel outputs (111a-a to f) of a first motion sensing module (MSM) (111a) and possible multiple channel outputs (111b-a to f) of second motion sensing module (MSM) (111b) that may be present in the signal stream (120) are shown in **FIG. 11A. FIG. 3B** shows exemplary channel outputs from a BCG (Lin y, Rot x) and a SCG (Lin z and Rot y) during a non-exercise period (N), and from an ECG derivation.

A motion sensing module (MSM) (111) detects bodily vibrations induced by cardiovascular activity within the body. The bodily vibrations originate from the heart, and are transmitted through the body *via* tissue structures, including the vasculature and the skeleton. The bodily vibrations hence demonstrate a certain pattern that repeats with each heartbeat. The bodily vibrations are detected by mechanical transference to the motion sensing module (MSM) (111). A motion sensing module (MSM) (111) is discrete. By discrete, it is meant that it occupies or detects movements from a discrete region of the body. Where there are multiple motion sensing modules (MSMs), they may be disposed at different discrete locations of the body. Typically, a motion sensing module (MSM) (111) comprises an exterior (rigid) housing that receives the bodily vibrations, which are transmitted to one or more movement sensors disposed in fixed relation to the housing.

Non-limiting examples of motion sensing module (MSM) (111) include the seismocardiograph (SCG) (114) and the ballistocardiograph (BCG) (112) described elsewhere herein.

The motion sensing module (MSM) (111) outputs one or more channel outputs (*e.g.*, 111a-a to f or 111b-a to f), each channel output reporting a different movement type (linear or rotation) and/or different direction (x, y, z).

The motion sensing module (MSM) (111) is configured for placement on the subject at a discrete location. The placement is typically on the skin, or over a material (*e.g.*, garment) in contact with the skin. **FIG. 1A** and **1B** show examples of discrete locations (a to s). Principle examples of discrete locations include around the centre of mass (or gravity) of the subject (*e.g.*, as used in a BCG), lumbar region (lordosis curvature, between the second and the third lumbar vertebrae) (*e.g.,* as used in a BCG), thorax (*e.g.,* as used in a SCG), sternum, upper sternum (manubrium) (*e.g.*, as used in a SCG), sternum body (corpus sternal) (*e.g.*, as used in a SCG). Further examples of discrete locations include head (top, back, forehead), neck, shoulder, arm, wrist, hand, hand digit, leg, thigh, calf ankle, foot (above or base), foot digit. Shown in **FIG. 1A** is a motion sensing module (MSM) (111) located in different exemplary discrete locations: a - head, b - cervical region, c - thoracic region, d - upper arm (left side shown), e - lumbar region (lordosis curvature), f - wrist (left side shown), g - hand digit (left side shown), h - thigh (right side shown), i - calf (right side shown), j - ankle (right side shown), k - base of foot (left side shown). Shown in **FIG. 1B** is a motion sensing module (MSM) (111) located in different exemplary discrete locations: l - forehead, m - shoulder, n - upper sternum (manubrium), o - sternum body (corpus sternal), p - wrist, q - thigh, r - calf, s - foot digit. Where a motion sensing module (MSM) (111) is shown exemplarily on only one side of the body (e.g., left), it is understood it may alternatively or additionally be placed on the other side (e.g., right).

A motion sensing module (MSM) (111) comprises one or more movement sensors for detection of body movements. The one or more movement sensors may be contained within the motion sensing module (MSM) (111) housing. A movement sensor detects linear or rotational movement. Each movement sensor may generate one or more channel outputs. The sampling rate may be between 40 Hz and 1000 Hz. The motion sensing module (MSM) may comprise a movement sensor that is an accelerometer for measurement of linear acceleration. The accelerometer may be a one-, two-, or three-dimensional accelerometer for measurement of linear acceleration in one, two, or three different directions (1, 2 or 3 linear degrees of freedom (1, 2, or 3DOF)). The motion sensing module (MSM) (111) may generate one, two, or three channel outputs depending on the number of degrees of freedom being measured. Preferably, the motion sensing module (MSM) (111) comprises a three-dimensional accelerometer for measurement of linear acceleration in three different directions (3 linear degrees of freedom (3DOF)); the three-dimensional accelerometer preferably generates three channel outputs (**FIG. 11A,** 111a-a to -c or 111b-a to -c), one for each linear dimension being measured

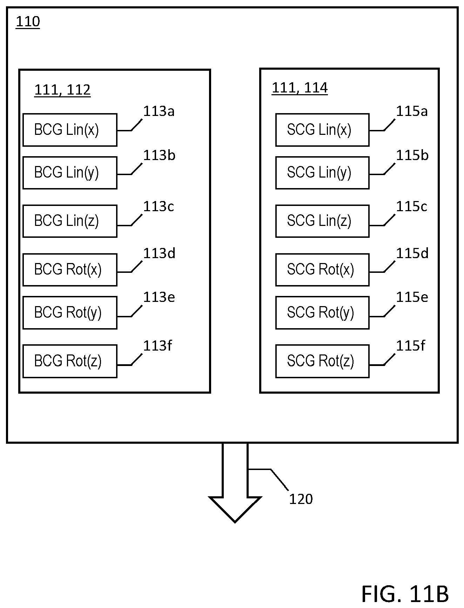

The motion sensing module (MSM) (111) may alternatively or additionally comprise a movement sensor that is a gyroscope for measurement of rotational movement. The gyroscope may be a one-, two-, or three-dimensional gyroscope for measurement of rotational movement in one, two, or three different directions (1, 2 or 3 linear degrees of freedom (1, 2, or 3DOF)). The motion sensing module (MSM) (111) may generate one, two, or three channel outputs depending on the number of degrees of freedom being measured. Preferably, the motion sensing module (MSM) (111) comprises a three-dimensional gyroscope for measurement of rotational movement around three different axes (3 linear degrees of freedom (3DOF)); the three-dimensional gyroscope preferably generates three channel outputs (FIG. **11B**, 111a-d to -f or 111b-d to -f), one for each rotational dimension being measured.

For motion sensing module (MSM) (111) measurements, an x-axis may be a lateral axis (transverse axis: from the subject's left to the subject's right); a y-axis may be a longitudinal axis (caudocranial, oriented from feet to head), a z-axis may be an antero-posterior axis (ventrodorsal) (see **FIGs. 1A and 1B**).

Preferably, the motion sensing module (MSM) (111) comprises at least two movement sensors, one being a one-, two-, or three- (preferably three) dimensional accelerometer and another being a one-, two-, or three- (preferably three) dimensional gyroscope. Preferably, the motion sensing module (MSM) (111) generates two or more channel outputs, one, two, or three (preferably three) from the accelerometer (**FIG. 11A,** 111a-a to -c or 111b-a to -c), and one, two, or three (preferably three) from the gyroscope (**FIG. 11A,** 111a-d to f or 111b-d to f).

The motion sensing module (MSM) may further comprise a means for attachment to the body of the subject, *e.g.,* a band or strap (*e.g.,* for attachment to chest, head or waist, wrist etc), an adhesive, a garment (*e.g.*, hat, sweat band, vest, glove, shorts, sock, shoe), or a wearable (*e.g.*, harness, watch, glasses, ring, hat). It may be incorporated into a fitness tracker (*e.g.*, chest band, watch, ankle strap).

The motion sensing module (MSM) may include an input for one or more electrocardiograph (ECG) electrodes for measurement of ECG signals. The motion sensing module (MSM) may include one or more interface modules for communication, for example, wireless or wired data transfer of the one or more channel outputs. Data transfer may be between the motion sensing module (MSM), and a smartphone, tablet, or other computing device. Data transfer may be between one motion sensing module (MSM) and one or more further motion sensing modules (MSM).

One example of a motion sensing module (MSM)(111) is a seismocardiograph (SCG)(114). The seismocardiograph (SCG) is configured for placement on the subject on the thorax, for instance, over the sternum, upper sternum (manubrium), or sternum body (corpus sternal). **FIG. 1A** shows placements of a seismocardiograph (114) on the subject in a discrete location that are the upper sternum (manubrium)(n) or sternum body (corpus sternal)(o). **FIG 1D** shows an exemplary placement of a seismocardiograph (114) on the subject in a discrete location that is the upper sternum (manubrium). **FIG. 2B** shows the seismocardiograph (114) as part of the sensor unit (110) and sending signals to the processing unit (160). The seismocardiograph measures movements of the heart, such as heart contraction and blood ejection into the aorta, such as, opening and closing of the aortic valve. The seismocardiograph (SCG) outputs one or more channel outputs, each channel output reporting a different movement (linear or rotation) and/or different direction (x, y, z).

The seismocardiograph (SCG) comprises one or more movement sensors for detection of bodily movements. A movement sensor detects linear or rotational movement. Each movement sensor may generate one or more channel outputs. The sampling rate may be between 40 Hz and 1000 Hz.

The seismocardiograph (SCG) may comprise a movement sensor that is an accelerometer for measurement of linear acceleration. The accelerometer may be a one-, two-, or three-dimensional accelerometer for measurement of linear acceleration in one, two, or three different directions (1, 2, or 3 linear degrees of freedom (1, 2, or 3DOF)). The seismocardiograph may generate one, two, or three channel outputs depending on the number of degrees of freedom being measured. Preferably, the seismocardiograph comprises a three-dimensional accelerometer for measurement of linear acceleration in three different directions (3 linear degrees of freedom (3DOF)); the three-dimensional accelerometer preferably generates output three channel outputs (**FIG. 11B**, 115a-c), one for each linear dimension being measured

The seismocardiograph (SCG) may alternatively or additionally comprise a movement sensor that is a gyroscope for measurement of rotational movement. The gyroscope may be a one-, two-, or three-dimensional gyroscope for measurement of rotational movement in one, two, or three different directions (1, 2 or 3 linear degrees of freedom (1, 2, or 3DOF)). The seismocardiograph may generate one, two, or three channel outputs depending on the number of degrees of freedom being measured. Preferably, the seismocardiograph comprises a three-dimensional gyroscope for measurement of rotational movement in three different axes (3 linear degrees of freedom (3DOF)); the three-dimensional gyroscope preferably generates three channel outputs (**FIG. 11,** 115d-f), one for each rotational dimension being measured.

For seismocardiograph measurements, an x-axis may be a lateral axis (transverse axis: from the subject's right to the subject's left); a y-axis may be a longitudinal axis (caudocranial, oriented from feet to head); a z-axis may be an antero-posterior axis (dorsoventral, from back to stomach) (see **FIG. 1B**).

Preferably, the seismocardiograph (SCG) comprises at least two movement sensors, one being a one-, two-, or three- (preferably three) dimensional accelerometer and another being a one-, two-, or three- (preferably three) dimensional gyroscope. Preferably, the seismocardiograph generates two or more channel outputs, one, two, or three (preferably three) from the accelerometer (**FIG. 11B,** 115a-c), and one, two, or three (preferably three) from the gyroscope (**FIG. 11B**, 115d-f).

The seismocardiograph may further comprise a means for attachment to the body of the subject, *e.g.*, mounting to a bodily-worn belt, adhesive gel pad. The seismocardiograph may include an input for one or more electrocardiograph (ECG) electrodes for measurement of ECG signals. The seismocardiograph may include one or more interface modules for communication, for example, wireless or wired data transfer of the one or more channel outputs. Data transfer may be between the seismocardiograph and a smartphone, tablet, or other computing device. Data transfer may be between the seismocardiograph and the ballistocardiograph.

One example of a motion sensing module (MSM)(111) is a ballistocardiocardiograph (BCG)(112). The ballistocardiograph (BCG) is configured for placement on the subject at or around the centre of mass (or gravity) the subject. **FIG. 1B** shows placements of a ballistocardiograph (112) on the subject in a discrete location that is the back lumbar region (lordosis curvature)(e). **FIG. 1C** shows an exemplary placement of a ballistocardiograph (112) on the subject at a discrete location that is the lordosis curvature of the back (between the second and the third lumbar vertebrae). The ballistocardiograph measures global body movements. The ballistocardiograph (BCG) outputs one or more channel outputs (113a-f), each channel output reporting a different movement type (linear or rotation) and/or different direction (x, y, z).

The ballistocardiograph (BCG) comprises one or more movement sensors for detection of body movements. A movement sensor detects linear or rotational movement. Each movement sensor may generate one or more channel outputs. The sampling rate may be between 40 Hz and 1000 Hz. The ballistocardiograph (BCG) may comprise a movement sensor that is an accelerometer for measurement of linear acceleration. The accelerometer may be a one-, two-, or three-dimensional accelerometer for measurement of linear acceleration in one, two, or three different directions (1, 2 or 3 linear degrees of freedom (1, 2, or 3DOF)). The ballistocardiograph may generate one, two, or three channel outputs depending on the number of degrees of freedom being measured. Preferably, the ballistocardiograph comprises a three-dimensional accelerometer for measurement of linear acceleration in three different directions (3 linear degrees of freedom (3DOF)); the three-dimensional accelerometer preferably generates three channel outputs (**FIG. 11B,** 113a-c), one for each linear dimension being measured

The ballistocardiograph (BCG) may alternatively or additionally comprise a movement sensor that is a gyroscope for measurement of rotational movement. The gyroscope may be a one-, two-, or three-dimensional gyroscope for measurement of rotational movement in one, two, or three different directions (1, 2 or 3 linear degrees of freedom (1, 2, or 3DOF)). The ballistocardiograph may generate one, two, or three channel outputs depending on the number of degrees of freedom being measured. Preferably, the ballistocardiograph comprises a three-dimensional gyroscope for measurement of rotational movement around three different axes (3 linear degrees of freedom (3DOF)); the three-dimensional gyroscope preferably generates three channel outputs (**FIG. 11B,** 113d-f), one for each rotational dimension being measured.

For ballistocardiograph (BCG) measurements, an x-axis may be a lateral axis (transverse axis: from the subject's left to the subject's right); a y-axis may be a longitudinal axis (caudocranial, oriented from feet to head), a z-axis may be an antero-posterior axis (ventrodorsal, from stomach to back) (see **FIG. 1A**).

Preferably, the ballistocardiograph (BCG) comprises at least two movement sensors, one being a one-, two-, or three- (preferably three) dimensional accelerometer and another being a one-, two-, or three- (preferably three) dimensional gyroscope. Preferably, the ballistocardiograph generates two or more channel outputs, one, two, or three (preferably three) from the accelerometer (**FIG. 11B,** 113a-c), and one, two, or three (preferably three) from the gyroscope (**FIG. 11B**, 113d-f).

The ballistocardiograph (BCG) may further comprise a means for attachment to the body of the subject, *e.g.*, mounting to a bodily-worn belt, adhesive gel pad. The ballistocardiograph may include an input for one or more electrocardiograph (ECG) electrodes for measurement of ECG signals. The ballistocardiograph may include one or more interface modules for communication, for example, wireless or wired transfer of the one or more channel outputs. Data transfer may be between the ballistocardiograph and a smartphone, tablet, or other computing device.

The sensor unit (110) may be configured to generate a stream of signals (120) from the seismocardiograph (SCG) and optionally from the ballistocardiograph (BCG). The sensor unit (100) signal stream (120) may contain:

- one or more channel outputs (115a-c) from one or more accelerometer axes of the SCG (3 linear (x, y, z) axes); and/or
- one or more channel outputs (115d-f) from one or more gyroscope axes of the SCG (3 rotational (x, y, z) axes); and/or
- one or more channel outputs (113a-c) from one or more accelerometer axes of the BCG (3 linear (x, y, z) axes); and/or
- one or more channel outputs (113d-f) from one or more gyroscope axes of the BCG (3 rotational (x, y, z) axes).

The sensor unit (100) signal stream (120) may contain channel outputs (115b) from the y-linear axis accelerometer axis of seismocardiograph (SCG). The sensor unit (100) signal stream (120) may contain channel outputs (115a-c) from the three accelerometer axes of ballistocardiograph (BCG) (all 3 linear (x, y, z) axes).

The sensor unit (100) signal stream (120) may contain channel outputs (113b) from the y-linear axis accelerometer axis of ballistocardiograph (BCG). The sensor unit (100) signal stream (120) may contain channel outputs (113a-c) from the three accelerometer axes of ballistocardiograph (BCG) (all 3 linear (x, y, z) axes).

It has been found that cardiorespiratory fitness measurement, in particular, the $VO_2$ max value can be determined from as little as one seismocardiograph (SCG) axis of movement (e.g., y-linear axis, one channel output (115b)), or one ballistocardiograph (BCG) axis of movement (e.g., y-linear axis, one channel output (113b)), however, the more axes of movement that can be taken into account the more optimised the cardiorespiratory fitness measurement, in particular, the $VO_2$ max value.

The sensor unit (110) may comprise (only) one motion sensing module (MSM)(111), for convenience to the subject. For ease of wearing, the sensor unit (110) may comprise (only) the seismocardiograph, SCG (114). In such case, the seismocardiograph (SCG) comprises at least one movement sensor; the movement sensor may be a one-, two-, or three-(preferably three) dimensional accelerometer, or a one-, two-, or three- (preferably three) dimensional gyroscope.

The sensor unit (110) may comprise both the ballistocardiograph, BCG, and the seismocardiograph, SCG. In such case, the ballistocardiograph (BCG) comprises at least one movement sensor being a one-, two-, or three- (preferably three) dimensional accelerometer, or a one-, two-, or three- (preferably three) dimensional gyroscope. The seismocardiograph (SCG) may comprise at least one movement sensor being a one-, two-, or three- (preferably three) dimensional accelerometer or a one-, two-, or three- (preferably three) dimensional gyroscope. The sensor unit (110) may generate two or more channel outputs:

- one, two, or three (preferably three) from the BCG accelerometer, and/or one, two, or three (preferably three) from the BCG gyroscope, and/or
- one, two, or three (preferably three) from the SCG accelerometer, and/or one, two, or three (preferably three) from the SCG gyroscope.

One example of a sensor unit (110) comprising both SCG and the BCG is a "Kino" created by HeartKinetics; Gosselies, Belgium. It is made of two small modules that independently collect the SCG and BCG signals of the subjects together with a 1-lead ECG (one lead contains of electrodes with opposite polarity).

The processing unit (160) comprises circuitry configured performing the method of the invention. Typically, the circuitry comprises a processor and a memory. The processing unit (160) may be implemented in a computing device such as a desktop PC, laptop, dedicated programmable controller, as a collection of connected computing devices. The processing unit may be provided in part or entirely by a processor and a memory disposed within a housing of the sensor unit (110), or ballistocardiograph (112) or seismocardiograph (114), or within a housing of a separate unit. The processing unit (160) may be configured for performing one of more of the methods, or parts thereof, as described herein.

The processing unit (160) contain or be part of a standard computer system such as an Intel Architecture IA-32 based computer system 2, and may implement programming instructions of one or more software modules stored on non-volatile (e.g., hard disk or solidstate drive) storage associated with the corresponding computer system. However, it will be apparent that at least some of the steps of any of the described processes could alternatively be implemented, either in part or in its entirety, as one or more dedicated hardware components, such as gate configuration data for one or more field programmable gate arrays (FPGAs), or as application-specific integrated circuits (ASICs), for example.

A kinetic energy data stream (KEDS) is determined for each non-exercise period (N) from the signal stream (120). The kinetic energy data stream (KEDS) is determined from the sensor unit (100) signal stream (120) on one or more output channels. The determination of KEDS for each non-exercise period (N) is shown schematically in **FIGs. 4, 5, 7, 9**. The kinetic energy data stream (KEDS) may be determined in real-time while the subject is in a non-exercise period (N) of an exercise session (E), or later from a recording of the sensor unit (100) signal stream (120) of an exercise session (E). The kinetic energy data stream (KEDS) comprises a plurality of discrete kinetic energy values (DKEV), each discrete kinetic energy value (DKEV) corresponding to a different time point or block of the non-exercise period (N).

The kinetic energy data stream (KEDS) may be determined from one or more of:

- linear kinetic energy along one or more axes (*e.g.* x, y, or z);
- rotational kinetic energy around one or more axes (*e.g.* x, y, or z);
- one or more parameter(s) obtained from the sensor unit (110) correlated to the linear kinetic energy and/or to the rotational kinetic energy.

The sensor unit (100) signal stream (120) may be transformed into a kinetic energy data stream (KEDS) according to the methods described in Hossein et al "Accurate Detection of Dobutamine-induced Haemodynamic Changes by Kino-Cardiography: A Randomised Double-Blind Placebo- Controlled Validation Study", Nature Scientific Reports (2019) 9:10479 (https://doi.org/10.1038/s41598-019-46823-3).

Linear kinetic energy ($K_{Lin}$) at time (t) for a single linear axis ((ax) which may be x, y, or z) may be determined according to the equation:

$$K_{Lin(ax)(t)} = 0.5\,[m \cdot v_{ax(t)}^2],$$

where

- $K_{Lin(ax)(t)}$ is the linear kinetic energy at time (t) along axis (ax) in Joules;
- $v_{ax(t)}$ is the linear velocity along axis (ax) measured by the MSM (*e.g.*, BCG or SCG) at time (t) in m/s;
- m is the mass of the subject in kg.

Where there are multiple channel outputs for multiple linear axis (x, y, and z), the channel outputs are summed, *viz*:

Linear kinetic energy ($K_{Lin}$) at time (t) for a multiple linear axis (x, y, and z) may be determined according to the equation:

$$K_{Lin(x,\,y,\,z)(t)} = 0.5\,[m((v_{x(t)}^2) + (v_{y(t)}^2) + (v_{z(t)}^2))]$$

- $K_{Lin(x,\,y,\,z)(t)}$ is the linear kinetic energy at time (t) along axes (x,y,z) in Joules;
- $v_{x(t)}$ is the linear velocity along axis (x) measured by the MSM (*e.g.*, SCG or BCG) at time (t) in m/s;
- $v_{y(t)}$ is the linear velocity along axis (y) measured by the MSM (*e.g.,* SCG or BCG) at time (t) in m/s;
- $v_{z(t)}$ is the linear velocity along axis (z) measured by the MSM (*e.g.*, SCG or BCG) at time (t) in m/s;
- m is the mass of the subject in kg.

Where two of the three axes are used, the axis not being used is removed from the equation.

Rotational kinetic energy ($K_{Rot}$) at time (t) around a single linear axis ((ax) which may be x, y, or z) may be determined according to the equation:

$$K_{Rot(ax)(t)} = 0.5\,[I_{(ax)} \cdot W_{(ax)(t)}^2],$$

where:

- $K_{Rot(ax)(t)}$ is the rotational kinetic energy at time (t) around axis (ax) in Joules;
- $I_{(ax)}$ is the moment of inertia around axis (ax) in kg.m$^2$. The moment of inertia may be that of the sensor unit, or that of the body. A model of human body moments of inertia is calculated at the centre-of-mass along the anatomical axis (I(ax)) that takes into account the height and weight of a subject (see also Matsuo, A., Ozawa, H., Goda, K. & Fukunaga, T. Moment of inertia of whole body using an oscillating table in adolescent boys. J. Biomech. 28, 219-223 (1995).)
- $W_{(ax)(t)}$ is the angular velocity around axis (ax) measured by the MSM (*e.g.*, SCG or BCG) at time (t) in rad/s.

Where there are multiple channel outputs for rotations around multiple linear axis (x, y, and z), the channel outputs are summed, *viz:*
Rotational kinetic energy ($K_{Rot}$) at time (t) around multiple linear axes (x, y, and z) may be determined according to the equation:

$$K_{Rot(x,y,z)(t)} = 0.5 \left[ I_{(x)} (W_{(x)(t)})^2 + I_{(y)} (W_{(y)(t)})^2 + I_{(z)} (W_{(z)(t)})^2 \right]$$

- $K_{Rot(x,y,w)(t)}$ is the rotational kinetic energy at time (t) around axes (x,y,z) in Joules;
- $I_{(x)}, I_{(x)}, I_{(x)}$ are the moment of inertia around axis (x), (y), (z) respectively in kg.m$^2$. The moments of inertia may be those of the sensor unit, or those of the body. A model of human body moments of inertia is calculated at the centre-of-mass along the anatomical axes (I(x), I(y), I(z)) that takes into account the height and weight of a subject (see also Matsuo, A., Ozawa, H., Goda, K. & Fukunaga, T. Moment of inertia of whole body using an oscillating table in adolescent boys. J. Biomech. 28, 219-223 (1995).)
- $W_{(x)(t)}, W_{(y)(t)}, W_{(z)(t)}$, are the angular velocity around axis (x), (y), (z) respectively measured by the MSM (*e.g.*, SCG or BCG) at time (t) in rad/s.

Where two of the three axes are used, the axis not being is used is removed from the equation.
The kinetic energy data stream (KEDS) may be determined from one or more parameters obtained from the sensor unit (110) that is correlated to the linear kinetic energy and/or to the rotational kinetic energy (*i.e.* one or more correlated parameters).
An example of a correlated parameter is amplitude (also known as magnitude) of one or more channel outputs (*e.g.*, 111a-a to f or 111b-a to f) of the motion sensing module (MSM) (111) of the sensor unit (110). In particular, an example of a correlated parameter is the amplitude expressed in acceleration, velocity, displacements, or force. Another example is signal energy of one or more channel outputs (*e.g.*, 111a-a to f or 111b-a to f) of the motion sensing module (MSM) (111) of the sensor unit (110). In particular, an example of a correlated parameter is the signal energy expressed in acceleration, velocity, displacements or force.
The correlated parameter that is amplitude is a norm of a signal from one or more channel outputs (e.g., 111a-a to f or 111b-a to f) of the motion sensing module (MSM) (111) of the sensor unit (110). This signal may be an acceleration vector, a velocity vector, a displacement vector or a force vector. The units of acceleration vector are preferably m/s$^2$. The units of the velocity vector are preferably m/s. The units of the displacement vector are preferably m. The units of the force vector are preferably N (Newton).
The amplitude at time (t) for a single linear axis ((ax) which may be x, y, or z) for a VoM may be determined according to the equation:

$$A_{Lin(ax)(t)} = sqrt \left[ VoM_{ax(t)}{}^2 \right],$$

where

- VoM is the vector of movement that is one of linear acceleration vector, linear velocity vector, linear displacement vector or linear force vector;
- $A_{Lin(ax)(t)}$ is the amplitude of the VoM at time (t) along axis (ax) in the VoM unit;
- $VoM_{ax(t)}$ is the VoM along axis (ax) measured by the MSM (*e.g.*, SCG or BCG) at time (t) in the VoM unit.

Where there are multiple channel outputs for multiple linear axis (x, y, and z) for a VoM, the channel outputs are summed, *viz:*
The amplitude at time (t) for multiple linear axes (x, y, and z) for a VoM may be determined according to the equation:

$$A_{Lin(x, y, z)(t)} = sqrt \left[ \left( (VoM_{x(t)}{}^2) + (VoM_{y(t)}{}^2) + (VoM_{z(t)}{}^2) \right) \right],$$

where

- VoM is the vector of movement that is one of linear acceleration vector, linear velocity vector, linear displacement vector or linear force vector;
- $A_{Lin(x, y, z)(t)}$ is the amplitude of the VoM at time (t) along axes (x,y,z) in the VoM unit;
- $VoM_{x(t)}$ is the VoM along axis (x) measured by the MSM (*e.g.*, SCG or BCG) at time (t) in the VoM unit;
- $VoM_{y(t)}$ is the VoM along axis (y) measured by the MSM (*e.g.,* SCG or BCG) at time (t) in the VoM unit;
- $VoM_{z(t)}$ is the VoM along axis (z) measured by the MSM (*e.g.*, SCG or BCG) at time (t) in the VoM unit.

Where two of the three axes are used, the axis not being is used is removed from the equation.

The amplitude at time (t) around a single linear axis ((ax) which may be x, y, or z) for a VoM may be determined according to the equation:

$$A_{Rot(ax)(t)} = sqrt\ [VoM_{ax(t)}{}^2],$$

where

- VoM is the vector of movement that is one of rotational acceleration vector, rotational velocity vector, rotational displacement vector or rotational force vector;
- $A_{Rot(ax)(t)}$ is the amplitude of the VoM at time (t) around axis (ax) in the VoM unit;
- $VoM_{ax(t)}$ is the VoM around axis (ax) measured by the MSM (*e.g.*, SCG or BCG) at time (t) in the VoM unit.

Where there are multiple channel outputs for multiple linear axis (x, y, and z) for a VoM, the channel outputs are summed, *viz:*

The amplitude at time (t) around multiple linear axes (x, y, and z) for a VoM may be determined according to the equation:

$$A_{Rot(x, y, z)(t)} = \ sqrt[((VoM_{x(t)}{}^2) + (VoM_{y(t)}{}^2) + (VoM_{z(t)}{}^2))],$$

where

- VoM is the vector of movement that is one of rotational acceleration vector, rotational velocity vector, rotational displacement vector or rotational force vector;
- $A_{Rot(x, y, z)(t)}$ is the amplitude of the VoM at time (t) around axes (x,y,z) in the VoM unit;
- $VoM_{x(t)}$ is the VoM around axis (x) measured by the MSM (*e.g.*, SCG or BCG) at time (t) in the VoM unit;
- $VoM_{y(t)}$ is the VoM around axis (y) measured by the MSM *(e.g.,* SCG or BCG) at time (t) in the VoM unit;
- $VoM_{z(t)}$ is the VoM around axis (z) measured by the MSM (e.g., SCG or BCG) at time (t) in the VoM unit.

Where two of the three axes are used, the axis not being is used is removed from the equation.

The correlated parameter that is signal energy is an area under a squared amplitude (or magnitude) of a signal from one or more channel outputs (*e.g.*, 111a-a to f or 111b-a to f) of the motion sensing module (MSM) (111) of the sensor unit (110). This signal may be an acceleration vector, a velocity vector, a displacement vector or a force vector. The units of acceleration vector are preferably m/s². The units of the velocity vector are preferably m/s. The units of the displacement vector are preferably m. The units of the force vector are preferably N (Newton).

The signal energy at time (t) for a single linear axis ((ax) which may be x, y, or z) for a VoM may be determined according to the equation:

$$E_{Lin(ax)(t)} = \ integral(A_{Lin(ax)(t)}{}^2),$$

- $E_{Lin(ax)(t)}$ is the energy of the VoM at time (t) along axis (ax) in the VoM unit;
- $A_{Lin(ax)(t)}$ is the amplitude of the VoM at time (t) along axis (ax) in the VoM unit;
- VoM is the vector of movement that is one of linear acceleration linear vector, linear velocity vector, linear displacement vector or linear force vector.

The signal energy at time (t) for multiple linear axes (x, y, and z) of a VoM may be determined according to the equation:

$$E_{Lin(x,\,y,\,z)(t)} = \text{integral}(A_{Lin(x,\,y,\,z)(t)}{}^2),$$

where

- $E_{Lin(x,\,y,\,z)(t)}$ is the energy of the VoM at time (t) along axes (x,y,z) in the VoM unit;
- $A_{Lin(x,\,y,\,z)(t)}$ is the amplitude of the VoM at time (t) along axes (x,y,z) in the VoM unit;
- VoM is the vector of movement that is one of linear acceleration vector, linear velocity vector, linear displacement vector or linear force vector.

Where two of the three axes are used, the axis not being is used is removed from the equation.

The signal energy at time (t) around a single linear axis ((ax) which may be x, y, or z) for a VoM may be determined according to the equation:

$$E_{Rot(ax)(t)} = \text{integral}(A_{Rot(ax)(t)}{}^2),$$

- $E_{(ax)(t)}$ is the energy of the VoM at time (t) around axis (ax) in the VoM unit;
- $A_{Rot(ax)(t)}$ is the amplitude of the VoM at time (t) around axis (ax) in the VoM unit;
- VoM is the vector of movement that is one of rotational acceleration vector, rotational velocity vector, rotational displacement vector or rotational force vector;

The signal energy at time (t) around multiple linear axes (x, y, and z) of a VoM may be determined according to the equation:

$$E_{Rot(x,\,y,\,z)(t)} = \text{integral}(A_{Rot(x,\,y,\,z)(t)}{}^2),$$

where

- $E_{Rot(x,\,y,\,z)(t)}$ is the energy of the VoM at time (t) around axes (x,y,z) in the VoM unit;
- $A_{Rot(x,\,y,\,z)(t)}$ is the amplitude of the VoM at time (t) around axes (x,y,z) in the VoM unit;
- VoM is the vector of movement that is one of rotational acceleration vector, rotational velocity vector, rotational displacement vector or rotational force vector.

Where two of the three axes are used, the axis not being is used is removed from the equation.

A mean kinetic energy (MKE) that is a time-averaged kinetic energy value from a time window (W) of the kinetic energy data stream is obtained. The time window (W) is for a period of w seconds. The value of w is less than the duration of the non-exercise period (N). Typically, w is greater than a duration of 1 cardiac cycle of the subject. The value of w may be in a range of 2 to 10 seconds. The value of w is preferably the same during each non-exercise period (N). The value of w is preferably the same during each non-exercise period (N) and during the exercise session (E).

A mean kinetic energy (MKE) may be determined from a time window (W) of the kinetic energy data stream (KEDS) by any method to compute the mean of discrete kinetic energy values (DKEV) over a given time window. For instance, the mean kinetic energy (MKE) may be determined from an integration of the discrete kinetic energy values (DKEV) measured within the time window (W) of the kinetic energy data stream (KEDS). For instance, the mean kinetic energy (MKE) may be determined from a time average of the discrete kinetic energy values (DKEV) measured within the time window (W) of the kinetic energy data stream (KEDS).

A period of mean kinetic energies (PMKEs) is determined within each non-exercise period (N), by determining each mean kinetic energy (MKE) of the multiple at a different starting time points within the non-exercise period (N). Some periods of mean kinetic energies (PMKEs) may overlap but have different starting time points. Preferably, a first mean kinetic energy (MKE) is determined at the start of the non-exercise period (N), a last mean kinetic energy (MKE) is determined at the end of the non-exercise period (N), and intermediate mean kinetic energies (MKEs) are determined at regular intervals between the first and last MKEs; this plurality of MKEs constitute the period of mean kinetic energies PMKEs. The time difference between consecutive time windows (W) and hence mean kinetic energy (MKE) may be constant or different within a non-exercise period (N). For instance, consecutive time windows (W) may differ in starting time of 0.5 to 2 seconds.

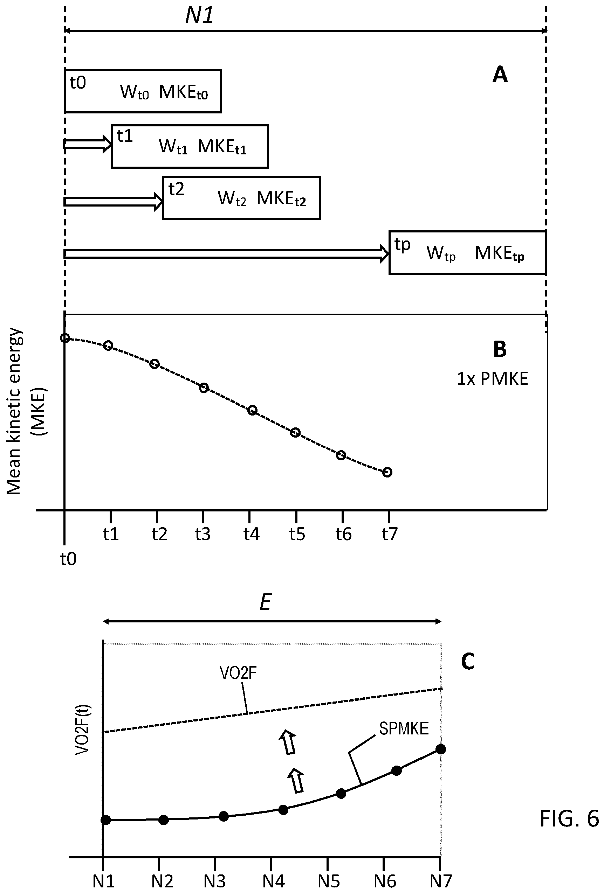

An example of a plurality of mean kinetic energies (MKEs) (MKEt0, MKEt1, MKEt2, MKEtp) determined from different time windows (Wt0, Wt1, Wt2, Wtp) during a non-exercise period (N) is shown in **FIG. 6A.** The starting time (t0, t1, t2, tp) for each time window (Wt0, Wt1, Wt2, Wtp) in incrementally larger than the starting time (t1, t2, tp) for a previous time window (Wt1, Wt2, Wtp). Values of MKE at different time points (t0 to t7) within a non-exercise period (N) are shown as a graph in **FIG. 6B,** the plurality of MKE values forming one period of mean kinetic energies (PMKE). The oxygen consumption rate ($VO_2$) for the subject can be determined from a set of periods of mean kinetic energies (SPMKE) for the exercise session (E) (**FIG. 6C**).

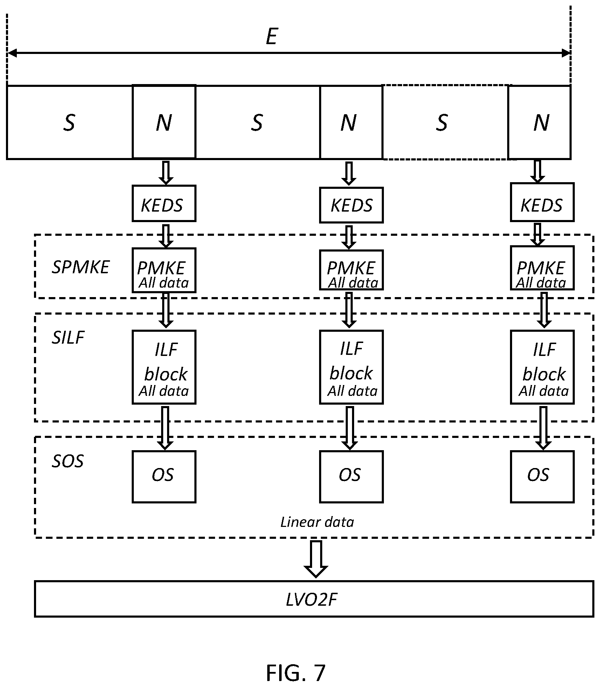

A plurality of PMKEs (periods of mean kinetic energy) obtained during the course of the exercise session (E), one PMKE per non-exercise period, is known as set of PMKEs (SPMKE). The determination of PMKEs for each non-exercise period (N) within the exercise session (E) (the SPMKE) is shown schematically in **FIGs. 7** and **9.**

The cardiorespiratory fitness measurement of the subject can be determined from the set of PMKEs (SPMKE). The SPMKE is indicative of the oxygen consumption rate ($VO_2$) over time for the exercise session (E). From the oxygen consumption rate ($VO_2$) over time, the $VO_2$ max value can be determined, which as mentioned elsewhere is one measurement of cardiorespiratory fitness.

In particular, the SPMKE may be transformed into a linear $VO_2$ function (LVO2F) for the subject, which $VO_2$ function (LVO2F) is representative of the oxygen consumption rate ($VO_2$) as a function of time of the subject during the exercise period. The determination of LVO2F from the SPMKE within the exercise session (E) is shown schematically in **FIGs. 4, 5, 7** and **9; FIGs. 7** and **9** show additional transformational steps of the SPMKE in detail.

In order to transform the SPMKE into the LVO2F, each PMKE of the set of PMKEs (SPMKE) is converted into a scalar value (single value) representative of the non-exercise period (N), wherein the scalar value is an order statistic. Thus, a set of order statistic (SOS) for the exercise session (E) is obtained, and from the evolution over time of the order statistics (OSs) of the SOS, the linear $VO_2$ function (LVO2F) for the subject can be determined. The determination of LVO2F from the SPMKE within the exercise session (E) using a set of order statistic (SOS) is shown schematically in **FIG. 5.**

An order statistic (OS) is determined for each non-exercise period (N). An order statistic (OS) may be a median, or another quantile (*e.g.*, 1st and 3rd quartile).

To generate the order statistic (OS) for each non-exercise period (N), each PMKE of the set of PMKEs (SPMKE) is converted into a scalar value (single value). This conversion may be performed:

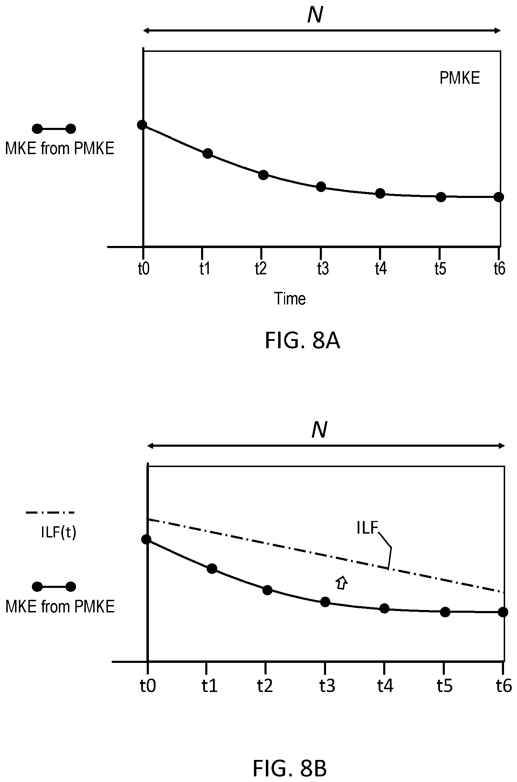

- indirectly on the PMKE (by transforming the MKE values within the PMKE first into another function, such as an intermediate linear function) (**FIGs. 7, 8A** to **8D**), or
- directly on the PMKE (by value-ordering the MKE values within the PMKE) (**FIGs. 9, 10A** to **10E**)

In the case of transforming indirectly the PMKE into an order statistic (OS), the period of mean kinetic energies (PMKEs) within a non-exercise period (N) is transformed into an intermediate linear function (ILF); this may be achieved using a scaling law. For instance, each mean kinetic energy (MKE) from the period of mean kinetic energies (PMKEs) may be plotted on a graph (y-axis: MKE, x-axis: time), to which a curve can be fitted. The fitted curve is transformed into an intermediate straight line (the intermediate linear function) by the scaling law. The intermediate linear function may be expressed graphically or as a mathematical formula. The scaling law may be any function that linearises a curve. In particular, the scaling law may use a cubic root of the mean kinetic energy (MKE). In particular, the scaling law may use the heart rate (HR) of the subject. One example of a scaling law comprises the equation HR * CubicRoot(MKE/HR).

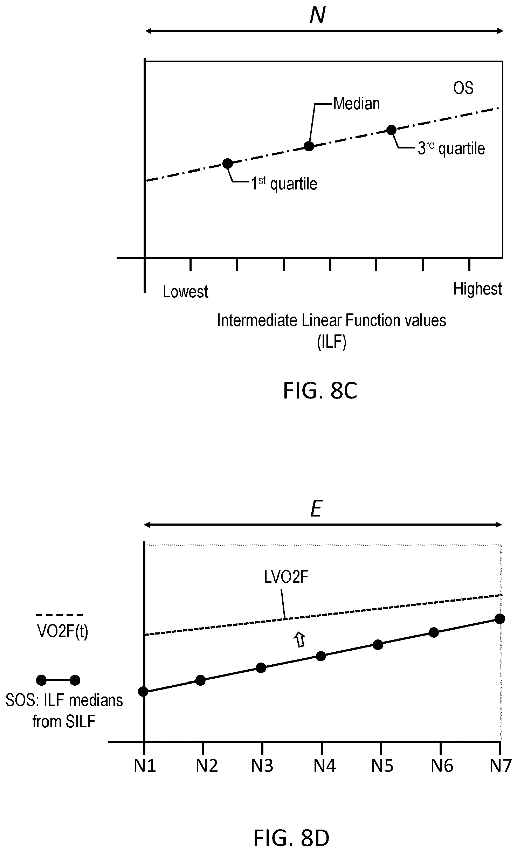

**FIG. 8A** shows, for non-exercise period (E), a graph of MKE values (y-axis) from a PMKE over time (x-axis, at time intervals (t0 to t6) within a non-exercise session (N)); in **FIG. 8B,** the period of mean kinetic energies (PMKEs) has been transformed into an intermediate linear function (ILF) by the scaling law (y-axis: ILF(t), x-axis: time). Then, intermediate linear functions is populated by a block of ILF values for a non-exercise period. The ILF values are arranged in value order (*e.g.,* largest to the smallest or *vice versa*). From the value-ordered ILF values, the median (mid-value), or quantile(s) can be determined. **FIG. 8C** shows an example of determining order statistics (OSs) - median value, 1st and 3rd quartiles - from value-ordered (lowest to highest) ILF values within the ILF block determined for a non-exercise period (N). An order statistic (OS) is determined for each non-exercise period (N).

In the case of transforming directly the PMKE into an order statistic, each mean kinetic energy (MKE) from the period of mean kinetic energies (PMKEs) within a non-exercise period (N) is arranged in value order (*e.g.,* largest to the smallest or *vice versa*). From the value-ordered mean kinetic energies (MKEs), the median (mid-value), or quantile(s) can be determined. **FIG. 10B** shows an example of determining order statistics (OSs) - median value, 1st and 3rd quartiles - from value-ordered (lowest to highest) mean kinetic energies (MKEs) within the period of mean kinetic energies (PMKEs) determined for a non-exercise period (N). An order statistic (OS) is determined for each non-exercise period (N).

The same order statistic (median or quantile) is determined for each non-exercise period (N), and at least some of these order statistics (OS) form a set of order statistics (SOS) for the exercise session (E). For example, the set of order statistics (SOS) may contain only median order statistics (and not a mix of median and quantile order statistics).

The set of order statistics (SOS) preferably contains only order statistics (OS) showing a linear evolution over time by the subject. The order statistics (OS) for inclusion in the set of order statistics (SOS) may be determined by including the first three order statistics (OS) related to the three first non-exercise periods (N) (excluding the very first measurement taken before the first exercise period), then progressively adding the following order statistics and evaluating if the new set of order statistics (SOS) has a better linear correlation coefficient (*e.g.*, Pearson Correlation Coefficient) than the previous one. The set of order statistics (SOS) is considered complete whenever the addition of a new order statistics results in a decrease of the linear correlation coefficient. Typically, the last (by time) order statistics (OS) are not included in the set of order statistics (SOS) because the subjects reach a point of non-linearity. Advantageously, measuring cardiorespiratory fitness, in particular, the $VO_2$ max based on a submaximal effort is more comfortable (less exhausting) for the subject. The cardiorespiratory fitness measurement, in particular, the $VO_2$ max can be determined from the set of order statistics (SOS). As elaborated elsewhere herein, cardiorespiratory fitness measurement, in particular, the $VO_2$ max can be determined from a set of order statistics (SOS) by transformation into a $VO_2$ function (VO2F) for the subject

In order to transform the SOS into the LVO2F, a proportionality function is employed. The proportionality function is applied to the evolution over time of the order statistics (OSs) of the SOS to arrive at the linear $VO_2$ function (LVO2F) for the subject using a proportionality function. The proportionality function is described in more detail below.

Once the linear $VO_2$ function (LVO2F) is obtained for the subject, the value on the linear VO2F curve at a point that corresponds to the actual (or estimated) maximal load is determined. This can be based on observation of a plateau in the order statistics (OS). Alternatively, it can be based on the point of the linear $VO_2$ function (LVO2F) corresponding to the actual (or estimated) maximal heart rate.

This estimated value may be corrected by a constant ('d'). The constant (d) corrects for recovery by the subject during the non-exercise period that would not arise if measurement has been made during an exercise session (S).

This transformation of the SOS into the LVO2F by the proportionality function may be performed

- directly on the SOS (**FIGs. 7, 8D**), or
- indirectly on the SOS (by first transforming the OS values within the SOS into another function, such as an intermediate linear function) (**FIGs. 9, 10D, 10E**).

In the case of transforming directly the SOS into the LVO2F, the proportionality function is applied to the SOS. For instance, the OS values of the SOS may be plotted on a graph (y-axis: OS (t), x-axis: time) as a first straight line, and the SOS is transformed into the LVO2F by the proportionality function. The $VO_2$ function (VO2F) may be expressed graphically or as a mathematical formula. In **FIG. 8D,** the set of order statistics (SOSs) for the exercise session (E) have been transformed into the $VO_2$ function (VO2F) by the proportionality function.

In the case of transforming indirectly the SOS into the LVO2F, the set of order statistics (SOS) may be first transformed into an intermediate linear function (ILF) for the exercise session (E); this may be achieved using a scaling law. For instance, the set of order statistics (SOS) may be plotted on a graph (y-axis: order statistic value, x-axis: time), to which a curve can be fitted. The fitted curve is transformed into a first straight line (the linear function) by the scaling law. The linear function may be expressed graphically or as a mathematical formula. **FIG. 10C** shows, for an exercise session (E), a graph of medians (y-axis) over time (x-axis, non-exercise periods (N1 to N7)); in **FIG. 10D,** the set of order statistics (SOS) has been transformed into a linear function (LF) by the scaling law (y-axis: ILF(t), x-axis: time). The scaling law may be any function that linearises a curve. In particular, the scaling law may use a cubic root of the order statistic (OS). In particular, the scaling law may use the heart rate (HR) of the subject. One example of a scaling law comprises the equation HR * CubicRoot(OS/HR).

Then, the intermediate linear function (ILF) is transformed into the VO2 function (VO2F) for the subject; this may be achieved using the proportionality function. The proportionality function is described in more detail below. The VO2 function (VO2F) is representative of the oxygen consumption of the subject as a function of time during the exercise session (E). For instance, the intermediate linear function (ILF) may be plotted on a graph (y-axis: LF (t), x-axis: time) as a first straight line, and the LF is transformed into a second straight line (VO2 function) by the proportionality function. The intermediate linear function (ILF) is transformed into the $VO_2$ function (second straight line) by the proportionality function. The VO2 function (VO2F) may be expressed graphically or as a mathematical formula. In **FIG. 10D,** the set of order statistics (SOSs) for the exercise session (E) have been transformed into a linear function (LF) by the scaling law, and the intermediate linear function (ILF) has been transformed into the VO2 function (VO2F) by the proportionality function (**FIG. 10E**).

As mentioned earlier, the set of order statistics (SOS) or intermediate linear function (LF) is transformed into a $VO_2$ function (VO2F) for the subject; this may be achieved using a proportionality function.

The proportionality function may include a step of multiplication by a constant (Q). The constant (Q) may be fixed for a subject; it may be different from one subject to another.

One example of a proportionality function comprises the equation Q(LF). One example of a function for determining the LVO2F is

$$y \ (VO2F) = Q(LF) + d$$

The value of the constant Q may be determined by calibrating the subject using an ergospirometry measurement. After this first measurement, the value of Q and additional VO2 max measurements may be consulted on the same subject. Alternatively, the value of Q based on parameters from the subjects (height, weight, body composition, cross-section area of the aorta, blood composition). The value of d, as mentioned elsewhere herein, corrects for recovery by the subject during the non-exercise period that would not arise if measurement has been made during an exercise session (S).

When it is mentioned that a kinetic energy data stream (KEDS) is determined for each non-exercise period (N) from the signal stream (120), it is understood that one or more kinetic energy data streams (KEDS) may be generated, depending on whether one or multiple channel outputs are being used.

For instance, where only one channel output (*e.g.*, BCG Lin(y), 113b) of the signal stream (120) is being used, only one kinetic energy data stream (KEDS) may be determined from the one channel (*e.g.*, BCG Lin(y), 113b). Where multiple (two or more) channel outputs (*e.g.*, BCG Lin(x), 113a; BCG Lin(y), 113b; BCG Lin(z), 113c) are being utilised, only one kinetic energy data stream (KEDS) may be determined by combining the channel outputs. For instance, one kinetic energy data stream (KEDS) that is a single BCG linear kinetic energy (KLin(BCG)) may be determined from a combination of BCG channel outputs BCG Lin(x), 113a; BCG Lin(y), 113b; BCG Lin(z), 113c. Other combinations of channel outputs are foreseen: combining KLin (BCG) with: KLin (SCG), and/or with KRot (BCG), and/or with KRot (SCG). It is foreseen that one kinetic energy data stream (KEDS) may be determined by combining any two of more channel outputs (113a-f; 115a-f). It is described elsewhere exemplary equations for generating a KEDS from one channel output or multiple channel outputs.

The subsequent steps of determining period of mean kinetic energy (PMKEs), order statistics, transformations using a scaling law and a proportionality function to arrive at the $VO_2$ function are hence performed using only one channel output (e.g. BCG Lin(y), 113b) from the sensor unit (100) signal stream (120).

Multiple kinetic energy data stream (KEDSs) may be determined from multiple (two or more) channel outputs (*e.g.*, BCG Lin(x), 113a; BCG Lin(y), 113b; BCG Lin(z), 113c). For example, one KEDS may be determined from each of the multiple channel outputs (*e.g.*, one KEDS for each of BCG Lin(x), 113a; BCG Lin(y), 113b; BCG Lin(z), 113c).

Where multiple KEDS are determined, the subsequent steps of determining periods of mean kinetic energies (PMKEs), order statistics, transformations using a scaling law and a proportionality function to arrive at the $VO_2$ function may be performed for each of the different channel outputs (113a-f; 115a-f), to arrive at a plurality of VO2Fs, one per channel output (113a-f; 115a-f). The steps after generating the plurality of VO2Fs may combine channel output transformations, for instance, by summing or averaging, to arrive to a single function or value from which the cardiorespiratory fitness measurement, in particular, the $VO_2$ max value can ultimately be calculated.

For instance, a plurality of VO2Fs (each generated from a different channel output (113a-f; 115a-f)) may be combined, e.g., by averaging (weighted or non-weighted), to arrive at a single VO2F. The cardiorespiratory fitness measurement, in particular, the $VO_2$ max value may be determined from the combined VO2F.

For instance, a plurality of the cardiorespiratory fitness measurements, in particular, of $VO_2$ max values (each generated from a different channel output (113a-f; 115a-f)) may be combined, e.g., by averaging (weighted or non-weighted), to arrive at a single cardiorespiratory fitness measurement, in particular, a single $VO_2$ max value.

The processing steps of the present method are performed *in vitro*.

The system (100) may include the sensor unit (110).

Further provided is a computer-implemented method for determining a cardiorespiratory fitness measurement, in particular, a maximal oxygen consumption, $VO_2$ max, of a subject comprising the steps carried out by the processing unit (160) as described elsewhere herein.

Further provided is a computer program or computer program product having instructions which when executed by a computing device or system cause the computing device or system to perform the computer-implemented method.

Further provided is a computer readable medium having stored thereon the computer program or computer program product.

Further provided is a data stream which is representative of the computer program or computer program product.

## Example

16 healthy subjects were recruited (8 males and 8 females). The demographics of the test subjects are presented in Table 1.

***Table 1****: Subject demographics represented by the median [first quartile; third quartile] for each group (males/ females).*

|  | Age (years) | Height (cm) | Weight (kg) | BMI (kg/m$^2$) |
|---|---|---|---|---|
| Males | 24 [23; 25] | 178 [173; 183] | 67 [66; 78] | 22.3 [20.8; 23.0] |

(continued)

| | Age (years) | Height (cm) | Weight (kg) | BMI (kg/m$^2$) |
|---|---|---|---|---|
| Females | 24 [23;27] | 167 [162; 173] | 59 [54; 62] | 20.9 [20.6; 21.7] |

These subjects performed a standard VO$_2$ max protocol using bicycle ergospirometry (ESt), as well as an exercise session (E1) as described herein including alternating exertion segments (S1) and non-exercise periods (N1). These two protocols are described in Table 2 and include Kino records in parallel with traditional analysis of gas exchanges.

*Table 2: Summary of the two protocols.*

| | Females | Males |
|---|---|---|
| *Standard protocol (ESt)* | • 60 s baseline Kino record<br>• 20 W increments every 60 s<br>• 30 s final Kino record | • 60 s baseline Kino record<br>• 30 W increments every 60 s<br>• 30 s final Kino record |
| *Test protocol (E1)* | • 60 s baseline Kino record<br>• 5 min warm-up at 80 W<br>• 30 s break with Kino record (N1)<br>• 10 W increase every 90 s (S1), each followed by a 30 s Kino record (N1)<br>• 30 s final Kino record | • 60 s baseline Kino record<br>• 5 min warm-up at 120 W<br>• 30 s break with Kino record (N1)<br>• 15 W increase every 90 s (S1), each followed by a 30 s Kino record (N1)<br>• 30 s final Kino record |

The results of VO$_2$ max given by ergospirometry (see Table 3) show that the test protocol (E1) gives results similar to the standard protocol (ESt). A Wilcoxon matched test gives indeed a p-value of 1.00, strongly supporting the equivalence of the two protocols.

*Table 3: Results of VO$_2$ max measured by ergospirometry in the protocols E0 and E1, for the male and female subjects. Results are given as median [first quartile; third quartile].*

| | Females | Males |
|---|---|---|
| *VO$_2$ max for ESt (l/min)* | 2.66 [2.49; 2.80] | 3.36 [2.52; 3.94] |
| *VO$_2$ max for E1 (l/min)* | 2.52 [2.41; 2.66] | 3.39 [2.88; 4.06] |

To estimate the value of VO$_2$ max based on the Kino records, we choose to focus on a kinetic energy data stream (KED) computed based on the linear axes of BCG:

$$K_{Lin(x, y, z)(t)} = 0.5 \left[ m((v_{x(t)}^2) + (v_{y(t)}^2) + (v_{z(t)}^2)) \right]$$

During each non-exercise period (N), the mean kinetic energy (MKE) was computed by integration of the KEDS signal over a window W of 5 seconds and normalisation by the length of this window. This mean kinetic energy (MKE) was computed by shifting the window every 1 second during a given non-exercise period (N) and resulted in a period mean kinetic energy (PMKE) for each non-exercise period (N).

The PMKE for each non-exercise period (N) was transformed into a linear function (LF) using the following scaling law:

$$LF = HR \cdot CubicRoot(MKE/HR)$$

where HR is the heart rate of the subject during the associated time-window W.

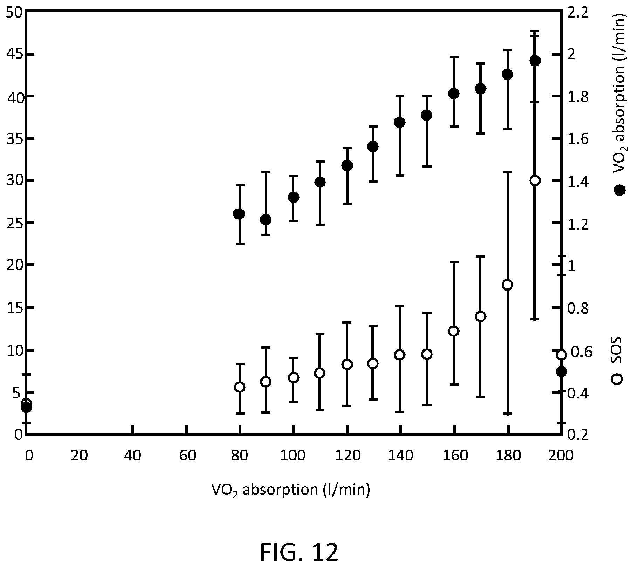

One LF was determined for each PMKE and hence for each non-exercise period (N1). An LF of a non-exercise period (N1) is populated by a block of LF values. From the block of LF values, an order statistic (OS) was extracted, given by the median of the block of LF values within the LF. For the exercise session (E1) a set of order statistics (SOS) was obtained, the SOS containing a plurality of order statics (medians), each order statics obtained from a block of LF values. An example of a set of order statistics (SOS) for an exercise session (E1) is shown in **FIG. 12.**

In **FIG. 12,** a linear (first part) part of the curve where the order statistics increase linearly with the workload, and a second part of the line (towards the end) where it becomes nonlinear with regard to workload is evident. The duration of the linear portion on the SOS curve of **FIG. 12** was found by considering the first three non-exercise periods (N1) (excluding baseline) and computing the Spearman linear correlation coefficient. Then, following non-exercise periods (N1) were progressively added and this Spearman linear correlation coefficient was computed again. The linear section was considered finished when a decrease in the Spearman coefficient was observed, as compared to the previous step.

For each of the SOS points that are in the linear section, the ratio between the median value of measured $VO_2$ and OS was determined. The median of these ratios gave the proportionality coefficient Q. The SOS curve was then transformed in a VO2F line, by multiplying each value by Q and fitting a line.

The value of the VO2F line at the maximal load reached by the subject was evaluated, which gave a preliminary evaluation of $VO_2$ max.

The value of $VO_2$ max was further optimised to correct for a slight underestimate, because it is based on measurements performed during non-exercise periods (N), during which the subject had some time to recover (depending on the duration of the non-exercise period and the level of fitness of the subject). A correction factor 'd' for each subject was determined, based on ergospirometry data. Alternatively, it was found that this correction factor was positively correlated to the value of the estimated $VO_2$ max, and may thus be approximated using a linear relationship.

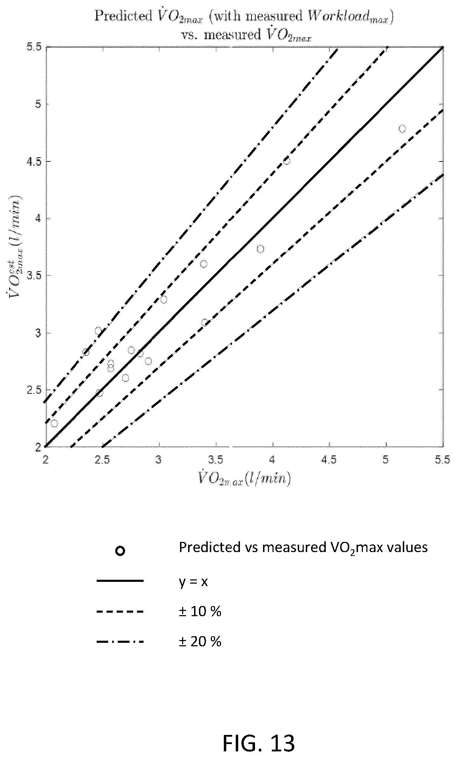

The results of the $VO_2$ max estimation with a known maximal workload and correction factor 'd' are given in Table 4. The estimates are very accurate, with a relative error that is mostly under 10%. A scatter plot of the estimated vs. measured value of $VO_2$ max is also given in **FIG. 13.** The points are indeed close to the identity line, and this is verified by computing the linear correlation coefficient: $R^2 = 0.8914$ (with a p-value of $6.63 \cdot 10^{-4}$).

*Table 4: Comparison of $VO_2$ max measured by ergospirometry and estimated by Kino, knowing the maximum workload achieved and the correction factor 'd'.*

| Subject | Measured VO$_2$ max (l/min) | Estimated VO$_2$ max (l/min) | Relative error (%) |
|---|---|---|---|
| GE | 2.57 | 2.72 | 6 |
| IJ | 2.70 | 2.60 | -4 |
| IU | 3.04 | 3.28 | 8 |
| LC | 2.90 | 2.75 | -5 |
| NB | 2.46 | 3.02 | 23 |
| OT | 2.75 | 2.85 | 3 |
| PG | 3.39 | 3.60 | 6 |
| RN | 2.47 | 2.47 | 0 |
| SD | 2.35 | 2.82 | 20 |
| TK | 2.83 | 2.82 | 0 |
| TN | 5.14 | 4.78 | -7 |
| TU | 2.57 | 2.69 | 5 |
| VF | 4.12 | 4.50 | 9 |
| VV | 2.07 | 2.21 | 7 |
| XF | 3.40 | 3.09 | -9 |
| YA | 3.89 | 3.73 | -4 |

We also verified the effect of missing input information, by estimating the maximum workload based on measured maximum heart rate and based on estimated maximum heart rate (220 - age). Each approximation leads to a decrease in the accuracy of the estimates, however the classification of the subjects according to their global fitness level remains very good (results not presented).

Additional experiments (results not presented) show that the proportionality coefficient Q is constant for a given subject.

**Claims**

1. System (100) for determining a cardiorespiratory fitness measurement of a subject (50), comprising a processing unit (160), the processing unit (160) configured to:

   - receive a stream of signals (120) outputted by a sensor unit (110) comprising one or more motion sensing modules (111), MSMs,
   - determine, from the signal stream (120), a kinetic energy data stream, KEDS, for each non-exercise period (N) of an exercise session (E) for gradually increasing the oxygen consumption, $VO_2$, of the subject, wherein the exercise session (E) comprises a plurality of alternating exertion segments (S) and non-exercise periods (N),
   - for each non-exercise period (N):

     - obtain a mean kinetic energy, MKE, that is a time-averaged kinetic energy value from a time window (W) of the kinetic energy data stream, KEDS;
     - determine a period of mean kinetic energies, PMKEs, containing multiple MKEs for different time windows (W) within the non-exercise period (N);

   - determine, from a set of PMKEs, SPMKE, containing multiple PMKEs within the exercise period (E), the cardiorespiratory fitness measurement of the subject.

2. The system (100) according to claim 1, wherein the cardiorespiratory fitness measurement of the subject is determined from the SPMKE by:

   - transforming the SPMKE into a linear VO2 function (LVO2F), representative of an oxygen consumption, $VO_2$, of the subject.

3. The system (100) according to claim 2, wherein the transformation of the SPMKE into the LVO2F, comprises a step of converting each PMKE of the SPMKE into a scalar value representative of the non-exercise period (N), wherein the scalar value is an order statistic.

4. The system (100) according to claim 3, wherein the conversion of each PMKE of the SPMKE into a scalar value is performed:

   - indirectly on the PMKE, by transforming the MKE values within the PMKE first into an intermediate linear function (ILF), and then by value-ordering the values within the ILF to obtain the order statistic, or
   - directly on the PMKE, by value-ordering the MKE values within the PMKE to obtain the order statistic.

5. The system (100) according to claim 4, wherein the same order statistic is determined for each non-exercise period (N), at least some of these order statistics (OS) form a set of order statistics, SOS, for the exercise session (E), and the SOS is transformed into the LVO2F of the subject.

6. The system (100) according to claim 5, wherein the transformation of the SOS into the LVO2F is performed:

   - directly on the SOS, or
   - indirectly on the SOS, by first transforming the order statistics (OS) values within the SOS into an intermediate linear function (ILF), and then transforming the intermediate linear function (ILF) into the LVO2F of the subject.

7. The system (100) according to any one of claims 1 to 6, wherein the order statistic (OS) is either a median or a quantile, and the order statistics (OS) within each set of order statistics (SOS) are either all medians or all quantiles.

8. The system (100) according to any one of claims 1 to 7, wherein the exercise session (E) brings the subject to a level of exhaustion, preferably to a level of full exhaustion.

9. The system (100) according to any one of claims 1 to 8, wherein the time window (W) is for a period of w seconds, and the value of w is greater than a duration of 1 cardiac cycle of the subject.

10. The system (100) according to claim 9, wherein the value of w is the same during each non-exercise period (N) and during the exercise session (E).

11. The system (100) according any one of claims 1 to 10 wherein the cardiorespiratory fitness measurement of the subject (50) is a $VO_2$ max measurement of the subject (50).

12. The system according to any one of claims 1 to 11, wherein one of the MSM (111) is a seismocardiograph (114), SCG, and optionally a further MSM (111) is a ballistocardiograph (112), BCG.

13. A computer-implemented method for determining a cardiorespiratory fitness measurement of a subject (50), comprising:

   - receiving a stream of signals (120) outputted by a sensor unit (110) comprising one or more motion sensing modules (111),,
   - determining, from the signal stream (120), a kinetic energy data stream, KEDS, for each non-exercise period (N) of an exercise session (E) for gradually increasing the oxygen consumption, $VO_2$, of the subject, wherein the exercise session (E) comprises a plurality of alternating exertion segments (S) and non-exercise periods (N),
   - for each non-exercise period (N):

      - obtaining a mean kinetic energy (MKE) that is a time-averaged kinetic energy value from a time window (W) of the kinetic energy data stream, KEDS;
      - determining multiple mean kinetic energies (MMKEs), for different time windows (W);
      - determining an order statistic (OS) from the multiple mean kinetic energies, (MMKEs)

   - determining a set of order statistics (SOS) containing at least some of the order statics (OS) determined from the plurality of non-exercise periods (N) of the exercise session (E);
   - determine from the set of order statistics (SOS), the cardiorespiratory fitness measurement of the subject.

14. The method according to claim 13, comprising a limitation as defined in any one of claims 2 to 11.

15. A computer program or computer program product having instructions which when executed by a computing device or system cause the computing device or system to perform the computer-implemented method of claim 13 or 14.


**Patentansprüche**

1. System (100) zum Bestimmen einer kardiorespiratorischen Fitnessmessung eines Subjekts (50), umfassend eine Verarbeitungseinheit (160), wobei die Verarbeitungseinheit (160) gestaltet ist zum:

   - Empfangen eines Stroms von Signalen (120), die von einer Sensoreinheit (110) ausgegeben werden, die ein oder mehrere Bewegungserfassungsmodule (111), MSMs, umfasst,
   - aus dem Signalstrom (120) Bestimmen eines Bewegungsenergie-Datenstroms, KEDS, für jeden Nichtbelastungszeitraum (N) einer Belastungssitzung (E) zum allmählichen Erhöhen des Sauerstoffverbrauchs, $VO_2$, des Subjekts, wobei die Belastungssitzung (E) eine Vielzahl von abwechselnden Belastungssegmenten (S) und Nichtbelastungszeiträumen (N) umfasst,
   - für jeden Nichtbelastungszeitraum (N):

      - Erhalten einer mittleren Bewegungsenergie, MKE, die ein zeitgemittelter Bewegungsenergiewert aus einem Zeitfenster (W) des Bewegungsenergie-Datenstroms, KEDS, ist;
      - Bestimmen eines Zeitraums von mittleren Bewegungsenergien, PMKEs, die mehrere MKEs für verschiedene Zeitfenster (W) innerhalb des Nichtbelastungszeitraums (N) enthalten;
      - Bestimmen, aus einem Satz von PMKEs, SPMKE, der mehrere PMKEs innerhalb des Belastuingszeitraums (E) enthält, der kardiorespiratorischen Fitnessmessung des Subjekts.

2. System (100) nach Anspruch 1, wobei die kardiorespiratorische Fitnessmessung des Subjekts aus dem SPMKE bestimmt wird durch:

   - Umwandeln des SPMKE in eine lineare VO2-Funktion (LVO2F), die für einen Sauerstoffverbrauch, $VO_2$, des Subjekts repräsentativ ist.

3. System (100) nach Anspruch 2, wobei die Umwandlung des SPMKE in den LVO2F einen Schritt des Umwandelns jedes PMKE des SPMKE in einen Skalarwert umfasst, der für den Nichtbelastungszeitraum (N) repräsentativ ist, wobei der Skalarwert eine Ordnungsstatistik ist.

4. System (100) nach Anspruch 3, wobei die Umwandlung jedes PMKE des SPMKE in einen Skalarwert durchgeführt wird:

- indirekt an dem PMKE, indem die MKE-Werte innerhalb des PMKE zuerst in eine lineare Zwischenfunktion (ILF) umgewandelt und dann die Werte innerhalb der ILF nach Wert geordnet werden, um die Ordnungsstatistik zu erhalten, oder
- direkt an dem PMKE, indem die MKE-Werte innerhalb der PMKE nach Wert geordnet werden, um die Ordnungsstatistik zu erhalten.

5. System (100) nach Anspruch 4, wobei die gleiche Ordnungsstatistik für jeden Nichtbelastungszeitraum (N) bestimmt wird, wobei wenigstens manche dieser Ordnungsstatistiken (OS) einen Satz von Ordnungsstatistiken, SOS, für die Belastungssitzung (E) bilden und der SOS in den LVO2F des Subjekts umgewandelt wird.

6. System (100) nach Anspruch 5, wobei die Umwandlung des SOS in den LVO2F durchgeführt wird:

- direkt auf dem SOS oder
- indirekt an dem SOS, indem zuerst die Werte der Ordnungsstatistik (OS) innerhalb des SOS in eine lineare Zwischenfunktion (ILF) umgewandelt werden und dann die lineare Zwischenfunktion (ILF) in die LVO2F des Subjekts umgewandelt wird.

7. System (100) nach einem der Ansprüche 1 bis 6, wobei die Ordnungsstatistik (OS) entweder ein Median oder ein Quantil ist und die Ordnungsstatistik (OS) innerhalb jedes Satzes von Ordnungsstatistiken (SOS) entweder alle Mediane oder alle Quantile sind.

8. System (100) nach einem der Ansprüche 1 bis 7, wobei die Belastungssitzung (E) das Individuum auf einen Grad der Erschöpfung, vorzugsweise auf einen Grad von völliger Erschöpfung, bringt.

9. System (100) nach einem der Ansprüche 1 bis 8, wobei das Zeitfenster (W) für einen Zeitraum von w Sekunden ist und der Wert von w größer als eine Dauer von 1 Herzzyklus des Subjekts ist.

10. System (100) nach Anspruch 9, wobei der Wert von w während jedes Nichtbelastungszeitraums (N) und während der Belastungssitzung (E) gleich ist.

11. System (100) nach einem der Ansprüche 1 bis 10, wobei die kardiorespiratorische Fitnessmessung des Subjekts (50) eine VO$_2$-max-Messung des Subjekts (50) ist.

12. System nach einem der Ansprüche 1 bis 11, wobei einer der MSM (111) ein Seismokardiograph (114), SCG, ist und gegebenenfalls ein weiterer MSM (111) ein Ballistokardiograph (112), BCG, ist.

13. Computerimplementiertes Verfahren zum Bestimmen einer kardiorespiratorischen Fitnessmessung eines Subjekts (50), umfassend:

- Empfangen eines Stroms von Signalen (120), die von einer Sensoreinheit (110) ausgegeben werden, die ein oder mehrere Bewegungserfassungsmodule (111) umfasst,
- aus dem Signalstrom (120) Bestimmen eines Bewegungsenergie-Datenstroms, KEDS, für jeden Nichtbelastungszeitraum (N) einer Belastungssitzung (E) zum allmählichen Erhöhen des Sauerstoffverbrauchs, VO$_2$, des Subjekts, wobei die Belastungssitzung (E) eine Vielzahl von abwechselnden Belastungssegmenten (S) und Nichtbelastungszeiträumen (N) umfasst,
- für jeden Nichtbelastungszeitraum (N):

- Erhalten einer mittleren Bewegungsenergie, MKE, die ein zeitgemittelter Bewegungsenergiewert aus einem Zeitfenster (W) des Bewegungsenergie-Datenstroms, KEDS, ist;
- Bestimmen mehrerer mittlerer Bewegungsenergien (MMKEs) für verschiedene Zeitfenster (W);
- Bestimmen einer Ordnungsstatistik (OS) aus den mehreren mittleren Bewegungsenergien (MMKEs);
- Bestimmen eines Satzes von Ordnungsstatistiken (SOS), der mindestens einige der Ordnungsstatistiken

**EP 4 475 755 B1**

(OS) enthält, die aus der Vielzahl von Nichtbelastungszeiträumen (N) der Übungssitzung (E) bestimmt werden;
- Bestimmen der kardiorespiratorischen Fitnessmessung des Subjekts aus dem Satz von Ordnungsstatistiken (SOS).

**14.** Verfahren nach Anspruch 13, umfassend eine Beschränkung nach einem der Ansprüche 2 bis 11.

**15.** Computerprogramm oder Computerprogrammprodukt mit Anweisungen, die, wenn durch eine Rechenvorrichtung oder ein Rechensystem ausgeführt, die Rechenvorrichtung oder das Rechensystem veranlassen, das computerimplementierte Verfahren nach Anspruch 13 oder 14 durchzuführen.

**Revendications**

**1.** Système (100) de détermination d'une mesure d'aptitude cardiorespiratoire d'un sujet (50) comprenant une unité de traitement (160), l'unité de traitement (160) étant configurée pour :

- recevoir un flux de signaux (120) délivrés par une unité de capteur (110) comprenant un ou plusieurs modules de capteur de mouvement (111), MSM,
- déterminer, à partir du flux de signaux (120), un flux de données d'énergie cinétique, KEDS, pour chaque période de non-exercice (N) d'une session d'exercice (E) pour augmenter progressivement la consommation d'oxygène $VO_2$, du sujet, où la session d'exercice (E) comporte une pluralité de segments d'exercice (S) et de périodes de non-exercice (N) alternés,
- pour chaque période de non-exercice (N) :

  - obtenir une énergie cinétique moyenne, MKE, qui est une valeur d'énergie cinétique moyennée dans le temps, à partir d'une fenêtre temporelle (W) du flux de données d'énergie cinétique, KEDS ;
  - déterminer une période d'énergies cinétiques moyennes, PMKE, contenant plusieurs MKE pour différentes fenêtres temporelles (W) à l'intérieur de la période de non-exercice (N) ;
  - déterminer, à partir d'un ensemble de PMKE, SPMKE, contenant plusieurs PMKE au cours de la période d'exercice (E), la mesure d'aptitude cardiorespiratoire du sujet.

**2.** Système (100) selon la revendication 1, dans lequel la mesure d'aptitude cardiorespiratoire du sujet est déterminée à partir du SPMKE par :

- transformation du SPMKE en une fonction $VO_2$ linéaire (LVO2F), représentative d'une consommation d'oxygène, $VO_2$, du sujet.

**3.** Système (100) selon la revendication 2, dans lequel la transformation du SPMKE en une LVO2F comprend une étape de conversion de chaque PMKE du SPMKE en une valeur scalaire représentative de la période de non-exercice (N), où la valeur scalaire est une statistique d'ordre.

**4.** Système (100) selon la revendication 3, dans lequel la conversion de chaque PMKE du SPMKE en une valeur scalaire est effectuée :

- indirectement sur le PMKE, en transformant les valeurs de MKE au sein du PMKE d'abord en une fonction linéaire intermédiaire (ILF), puis en classant par ordre les valeurs au sein de l'ILF pour obtenir la statistique d'ordre, ou
- directement sur le PMKE, en classant par ordre les valeurs MKE dans le PMKE pour obtenir la statistique d'ordre.

**5.** Système (100) selon la revendication 4, dans lequel la même statistique d'ordre est déterminée pour chaque période de non-exercice (N), au moins certaines de ces statistiques d'ordre (OS) forment un ensemble de statistiques d'ordre, SOS, pour la session d'exercice (E), et le SOS est transformé en la LVO2F du sujet.

**6.** Système (100) selon la revendication 5, dans lequel la transformation du SOS en la LVO2F est effectuée :

- directement sur le SOS, ou

- indirectement sur le SOS, en transformant d'abord les valeurs des statistiques d'ordre (OS) au sein du SOS en une fonction linéaire intermédiaire (ILF), puis en transformant la fonction linéaire intermédiaire (ILF) en la LVO2F du sujet.

7. Système (100) selon l'une quelconque des revendications 1 à 6, dans lequel la statistique d'ordre (OS) est soit une médiane soit un quantile, et les statistiques d'ordre (OS) dans chaque ensemble de statistiques d'ordre (SOS) sont soit toutes des médianes soit tous des quantiles.

8. Système (100) selon l'une quelconque des revendications 1 à 7, dans lequel la session d'exercice (E) amène le sujet à un niveau d'épuisement, de préférence à un niveau d'épuisement complet.

9. Système (100) selon l'une quelconque des revendications 1 à 8, dans lequel la fenêtre de temps (W) dure pendant une période de w secondes, et la valeur de w est supérieure à une durée de 1 cycle cardiaque du sujet.

10. Système (100) selon la revendication 9, dans lequel la valeur de w est la même pendant chaque période de non-exercice (N) et pendant la session d'exercice (E).

11. Système (100) selon l'une quelconque des revendications 1 à 10, dans lequel la mesure de l'aptitude cardiorespiratoire du sujet (50) est une mesure de la $VO_2$ max du sujet (50).

12. Système selon l'une quelconque des revendications 1 à 11, dans lequel l'un des MSM (111) est un sismocardiographe (114), SCG, et facultativement un autre MSM (111) est un ballistocardiographe (112), BCG.

13. Procédé, mis en œuvre par ordinateur, de détermination d'une mesure d'aptitude cardiorespiratoire d'un sujet (50), le procédé comprenant les étapes suivantes :

- recevoir un flux de signaux (120) délivrés par une unité de capteur (110) comprenant un ou plusieurs modules de capteur de mouvement (111),
- déterminer, à partir du flux de signaux (120), un flux de données d'énergie cinétique, KEDS, pour chaque période de non-exercice (N) d'une session d'exercice (E) pour augmenter progressivement la consommation d'oxygène $VO_2$, du sujet, où la session d'exercice (E) comporte une pluralité de segments d'exercice (S) et de périodes de non-exercice (N) alternés,
- pour chaque période de non-exercice (N) :

    - obtenir une énergie cinétique moyenne, MKE, qui est une valeur d'énergie cinétique moyennée dans le temps, à partir d'une fenêtre temporelle (W) du flux de données d'énergie cinétique, KEDS ;
    - déterminer de multiples énergies cinétiques moyennes (MMKE), pendant des fenêtres temporelles (W) différentes ;
    - déterminer une statistique d'ordre (OS) à partir des multiples énergies cinétiques moyennes, (MMKE)
    - déterminer un ensemble de statistiques d'ordre (SOS) contenant au moins une partie des statistiques d'ordre (OS) déterminées à partir de la pluralité de périodes de non-exercice (N) de la session d'exercice (E) ;
    - déterminer, à partir de l'ensemble de statistiques d'ordre (SOS), la mesure de l'aptitude cardiorespiratoire du sujet.

14. Procédé selon la revendication 13, comprenant une limitation telle que définie dans l'une quelconque des revendications 2 à 11.

15. Programme informatique ou produit programme informatique ayant des instructions qui, lorsqu'elles sont exécutées par un dispositif informatique ou un système informatique, amènent le dispositif informatique ou le système informatique à exécuter le procédé mis en œuvre par ordinateur selon la revendication 13 ou la revendication 14.

FIG. 1

FIG. 2A

FIG. 1 continued

FIG. 2B

FIG. 3

FIG. 4

FIG. 5

FIG. 6

FIG. 7

FIG. 8A

FIG. 8B

$N$

FIG. 8C

$E$

FIG. 8D

FIG. 9

FIG. 10A

FIG. 10B

FIG. 10C

FIG. 10D

FIG. 10E

FIG. 11A

FIG. 11B

FIG. 12

Predicted $\dot{V}O_{2max}$ (with measured $Workload_{max}$) vs. measured $\dot{V}O_{2max}$

| | |
|---|---|
| o | Predicted vs measured VO$_2$max values |
| —— | y = x |
| - - - - | ± 10 % |
| —·—·· | ± 20 % |

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3748641 A1

**Non-patent literature cited in the description**

- **HOSSEIN et al.** Accurate Detection of Dobutamine-induced Haemodynamic Changes by Kino-Cardiography: A Randomised Double-Blind Placebo- Controlled Validation Study. *Nature Scientific Reports*, 2019, vol. 9, 10479, https://doi.org/10.1038/s41598-019-46823-3

- **MATSUO, A.** ; **OZAWA, H.** ; **GODA, K.** ; **FUKUNAGA, T.** Moment of inertia of whole body using an oscillating table in adolescent boys.. *J. Biomech.*, 1995, vol. 28, 219-223
- **MATSUO, A** ; **OZAWA, H** ; **GODA, K.** ; **FUKUNAGA, T.** Moment of inertia of whole body using an oscillating table in adolescent boys.. *J. Biomech.*, 1995, vol. 28, 219-223